# EUROPEAN PATENT SPECIFICATION

(11) **EP 3 394 070 B1**
(45) Date of publication and mention of the grant of the patent: **02.08.2023**
(21) Application number: 15910869.5
(22) Date of filing: 14.12.2015
(51) Int. Cl.: C07D 513/14, A61K 31/4375, A61P 35/00

(54) **CRYSTALLINE FORMS OF QUINOLONE ANALOGS AND THEIR SALTS**
KRISTALLINE FORMEN VON CHINOLINANALOGA UND DEREN SALZEN
FORMES CRISTALLINES D'ANALOGUES DE QUINOLONE ET LEURS SELS

(43) Date of publication of application: 31.10.2018
(73) Proprietor: Senhwa Biosciences, Inc., New Taipei City 23143, (TW)
(72) Inventor: RYCKMAN David M., San Diego, CA 92130 (US); YU Iching Grace, San Diego, CA 92130 (US); LIU Hshiou-Ting, Milpitas, CA 95035 (US)
(74) Representative: Cooley (UK) LLP
(86) International application number: PCT/US2015/065443
(87) International publication number: WO 2017/105382

(56) References cited:
- WO-A1-2009/046383
- WO-A1-2012/123938
- WO-A1-2015/079411
- WO-A2-2007/022474
- RICHARD J BASTIN ET AL: "Salt Selection and Optimisation Procedures for Pharmaceutical New Chemical Entities", ORGANIC PROCESS RESEARCH AND DEVELOP, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]
- QUIN, JACLYN E. ET AL.: 'Targeting the nucleolus for cancer intervention' BIOCHIMICA ET BIOPHYSICA ACTA vol. 1842, 2014, pages 802 - 816, XP028599501
- DRYGIN, DENIS ET AL.: 'Targeting the nucleolus for cancer-specific activation of p53' DRUG DISCOVERY TODAY vol. 19, no. 3, 03 March 2014, pages 259 - 265, XP055391787

## Description

### FIELD OF THE DISCLOSURE

The present invention relates to crystalline forms of tetracyclic quinolone compounds or the salts and/or solvates of the tetracyclic quinolone compounds, pharmaceutical composition containing them, and methods of using them.

### BACKGROUND OF THE DISCLOSURE

A variety of tetracyclic quinolone compounds have been suggested to function by interacting with quadruplex-forming regions of nucleic acids and modulating ribosomal RNA transcription. *See,* for example, U.S. Patent Nos. 7,928,100 and 8,853,234. Specifically, the tetracyclic quinolone compounds can stabilize the DNA G-quadruplexes (G4s) in cancer cells and thereby induce synthetic lethality in cancer cells. Since treatment of cells with G4-stabilizing agents can lead to the formation of DNA double strand breaks (DSBs), DSB formation induced by G4-stabilizing ligand/agent (such as the tetracyclic quinolones) treatment would be more pronounced in cells genetically deficient in, or chemically inhibited in, repair pathways including both non-homologous end joining (NHEJ) and homologous recombination repair (HRR). Furthermore, the tetracyclic quinolone compounds selectively inhibit rRNA synthesis by Pol I in the nucleolus, but do not inhibit mRNA synthesis by RNA Polymerase II (Pol II) and do not inhibit DNA replication or protein synthesis. It is suggested that targeting RNA polymerase I (Pol I) to activate p53 through the nucleolar stress pathway may results in selective activation of p53 in tumor cells. The p53 protein normally functions as a tumor suppressor by causing cancer cells to self-destruct. Activating p53 to kill cancer cells is a well validated anticancer strategy and many approaches are being employed to exploit this pathway. Selective activation of p53 in tumor cells would be an attractive method of treating, controlling, ameliorating tumor cells while not affecting normal healthy cells. The aforementioned tetracyclic quinolones are disclosed in U.S. Patent Nos. 7,928,100 and 8,853,234.

Those skilled in the pharmaceutical arts understand that crystallization of an active pharmaceutical ingredient offers the best method for controlling important physiochemical qualities, such as stability, solubility, bioavailability, particle size, bulk density, flow properties, polymorphic content, and other properties. Thus, there is a need for crystalline forms of the tetracyclic quinolones and processes to produce such forms. These crystalline forms should be suitable for pharmaceutical use.

### SUMMARY OF THE DISCLOSURE

In one embodiment, the present invention provides a crystalline form of a tetracyclic quinolone compound I: 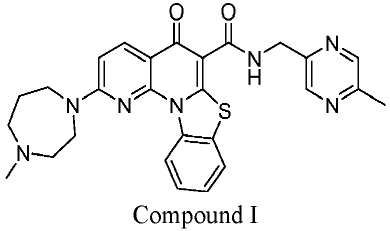 or a pharmaceutically acceptable salt thereof, wherein the crystalline form is hydrochloric acid salt of Compound I.

In a second embodiment, the present invention provides a composition comprising the above crystalline form of Compound I and at least one pharmaceutically acceptable carrier.

In a third embodiment, the present invention provides the above crystalline form or composition for use in treating or ameliorating cell proliferation disorder, wherein the cell proliferation disorder is cancer selected from: heme cancer, colorectum cancer, breast cancer, lung cancer, liver cancer, ovarian cancer, cervical cancer, Ewing's sarcoma, pancreatic cancer, cancer of the lymph nodes, colon cancer, prostate cancer, brain cancer, cancer of the head and neck, skin cancer, kidney cancer, or cancer of the heart.

In a fourth embodiment, the present invention provides the above crystalline form or composition for use in reducing or inhibiting cell proliferation, wherein the cells are in cancer cells selected from: heme cancer, colorectum cancer, breast cancer, lung cancer, liver cancer, ovarian cancer, cervical cancer, Ewing's sarcoma, pancreatic cancer, cancer of the lymph nodes, colon cancer, prostate cancer, brain cancer, cancer of the head and neck, skin cancer, kidney cancer, or cancer of the heart.

In a fifth embodiment, the present invention provides an HCl salt of Compound I.

Further embodiments of the invention are set out in the dependent claims.

### BRIEF DESCRIPTION OF THE DRAWINGS

**Figure 1** is a graph of an x-ray powder diffraction (XRPD) pattern of polymorph A of Compound I (free base).
**Figure 2** is a differential scanning calorimetry (DSC) thermogram of polymorph A of Compound I (free base).
**Figure 3** is a thermogravimetric analysis (TGA) thermogram of polymorph A of Compound I (free base).
**Figure 4** is an overlay of DSC and TGA thermograms of polymorph A of Compound I (free base).
**Figure 5** is a ¹H NMR spectrum of polymorph A of Compound I (free base).
**Figure 6A** is a microscopy picture of polymorph A of Compound I (free base) using crossed polarization filters and **Figure 6B** is a microscopy picture of polymorph A of Compound I (free base) with no polarization filters.
**Figure 7** is a graph of an XRPD pattern of polymorph C of Compound I (free base).
**Figure 8** is a DSC thermogram of polymorph C of Compound I (free base).
**Figure 9** is a ¹H NMR spectrum of polymorph C of Compound I (free base).
**Figure 10A** is a microscopy picture of polymorph C of Compound I (free base) using crossed polarization filters and **Figure 10B** is a microscopy picture of polymorph C of Compound I (free base) with no polarization filters.
**Figure 11** is a graph of an XRPD pattern of polymorph E of Compound I (free base).
**Figure 12** is a DSC thermogram of polymorph E of Compound I (free base).
**Figure 13** is a ¹H NMR spectrum of polymorph E of Compound I (free base).
**Figure 14A** is a microscopy picture of polymorph E of Compound I (free base) using crossed polarization filters and **Figure 14B** is a microscopy picture of polymorph E of Compound I (free base) with no polarization filters.

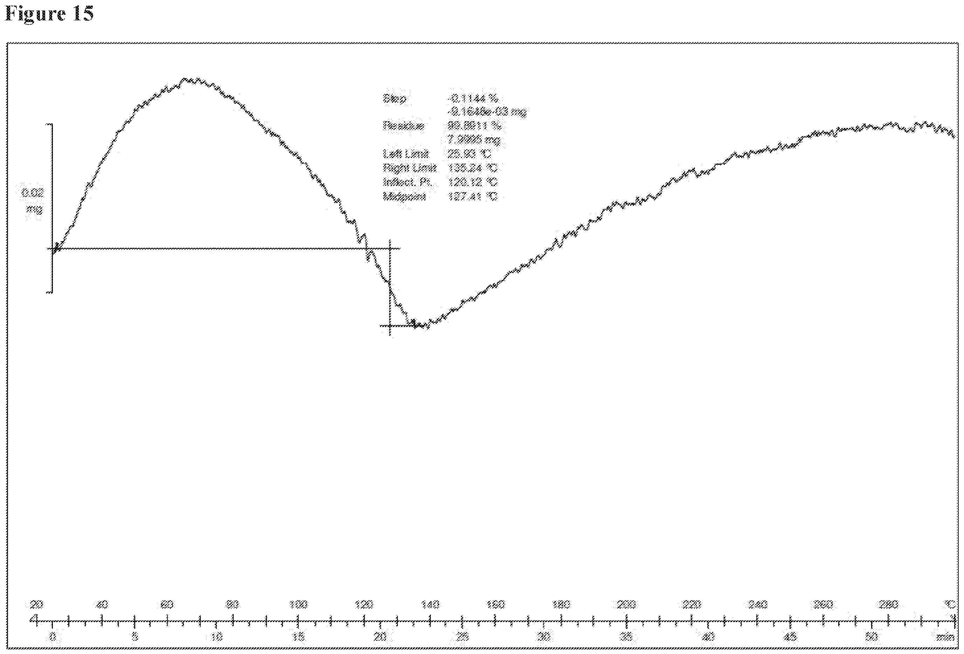

**Figure 15** is a thermogravimetric analysis (TGA) thermogram of polymorph E of Compound I (free base).
**Figure 16** is a graph of an XRPD pattern of polymorph G of Compound I (free base).
**Figure 17** is a DSC thermogram of polymorph G of Compound I (free base).
**Figure 18** is a ¹H NMR spectrum of polymorph G of Compound I (free base).
**Figure 19A** is a microscopy picture of polymorph G of Compound I (free base) using crossed polarization filters and **Figure 19B** is a microscopy picture of polymorph G of Compound I (free base) with no polarization filters.
**Figure 20** depicts the solubility of various polymorphs of Compound I (free base) in methanol and THF.
**Figure 21** is a graph of an XRPD pattern of HCl salt of Compound I.
**Figure 22** is a DSC thermogram of HCl salt of Compound I.
**Figure 23** is a ¹H NMR spectrum of HCl salt of Compound I.
**Figure 24** is a microscopy picture of HCl salt of Compound I using polarization filters.
**Figure 25** is a TGA thermogram of HCl salt of Compound I.
**Figure 26** is a graph of an XRPD pattern of maleic acid salt of Compound I.
**Figure 27** is a DSC thermogram of maleic acid salt of Compound I.
**Figure 28** is a ¹H NMR spectrum of maleic acid salt of Compound I.
**Figure 29** is a microscopy picture of maleic acid salt of Compound I using polarization filters.
**Figure 30** is a TGA thermogram of maleic acid salt of Compound I.
**Figure 31** is a graph of an XRPD pattern of fumaric acid salt of Compound I.
**Figure 32** is a DSC thermogram of fumaric acid salt of Compound I.
**Figure 33** is a ¹H NMR spectrum of fumaric acid salt of Compound I.
**Figure 34** is a microscopy picture of fumaric acid salt of Compound I using polarization filters.
**Figure 35** is a TGA thermogram of fumaric acid salt of Compound I.
**Figure 36** is a graph of an XRPD pattern of citric acid salt of Compound I.
**Figure 37** is a DSC thermogram of citric acid salt of Compound I.
**Figure 38** is a ¹H NMR spectrum of citric acid salt of Compound I.
**Figure 39** is a microscopy picture of citric acid salt of Compound I using polarization filters.
**Figure 40** is a TGA thermogram of citric acid salt of Compound I.
**Figure 41** is a graph of an XRPD pattern of L-malic acid salt of Compound I.
**Figure 42** is a DSC thermogram of L-malic acid salt of Compound I.
**Figure 43** is a ¹H NMR spectrum of L-malic acid salt of Compound I.
**Figure 44** is a microscopy picture of L-malic acid salt of Compound I using polarization filters.
**Figure 45** is a TGA thermogram of L-malic acid salt of Compound I.

### DETAILED DESCRIPTIONS OF THE DISCLOSURE

The present disclosure relates to crystalline forms of tetracyclic quinolone compounds, which stabilize G-quadruplexes (G4s) and/or inhibit Pol I, as well as crystalline forms of the salts and/or solvates of the tetracyclic quinolone compounds. These crystalline materials can be formulated into pharmaceutical compositions and used for treating disorders characterized by proliferation of cells.

### Definitions

It is to be understood that the terminology used herein is for the purpose of describing particular embodiments only and is not intended to be limiting.

Unless defined otherwise, all technical and scientific terms used herein have the same meaning as commonly understood to one of ordinary skill in the art to which the present application belongs. Although any methods and materials similar or equivalent to those described herein can be used in the practice or testing of the present application, representative methods and materials are herein described.

Following long-standing patent law convention, the terms "a", "an", and "the" refer to "one or more" when used in this application, including the claims. Thus, for example, reference to "a carrier" includes mixtures of one or more carriers, two or more carriers, and the like.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in the present specification and attached claims are approximations that can vary depending upon the desired properties sought to be obtained by the present application. Generally the term "about", as used herein when referring to a measurable value such as an amount of weight, time, dose, etc. is meant to encompass in one example variations of ±15% or ±10%, in another example ±5%, in another example ±1%, and in yet another example±0.1% from the specified amount, as such variations are appropriate to perform the disclosed method.

The term "compound(s) of the present disclosure" or "present compound(s)" refers to the crystalline form of 2-(4-Methyl-[1,4]diazepan-1-yl)-5-oxo-5H-7-thia-1,11b-diaza-benzo[c]fluorene-6-carboxylic acid (5-methyl-pyrazin-2-ylmethyl)-amide (Compound I) or isomers, salts, esters, N-oxides, or solvates thereof. Alternatively the above terms may refer to salt or solvate or both form of Compound I. The crystalline forms of Compound I described throughout the application including a crystalline form of any single isomer of Compound I, a mixture of any number of isomers of Compound I.

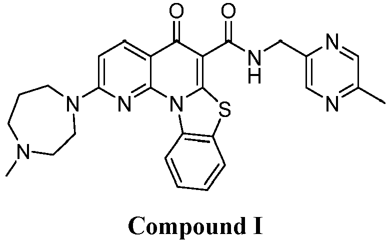

The term "co-administration" or "coadministration" refers to administration of (a) a crystalline form of Compound I, or a crystalline form of pharmaceutically acceptable salt, ester, solvate and/or prodrug of Compound I; and (b) one or more additional therapeutic agent and/or radio therapy, in combination, i.e., together in a coordinated fashion.

The term "isomer" refers to compounds having the same chemical formula but may have different stereochemical formula, structural formula, or special arrangements of atoms. Examples of isomers include stereoisomers, diastereomers, enantiomers, conformational isomers, rotamers, geometric isomers, and atropisomers.

"N-oxide", also known as amine oxide or amine-N-oxide, means a compound that derives from a compound of the present disclosure via oxidation of an amine group of the compound of the present invention. An N-oxide typically contains the functional group R₃N⁺-O⁻ (sometimes written as R₃N=O or R₃N→O).

Polymorphism can be characterized as the ability of a compound to crystallize into different crystal forms, while maintaining the same chemical formula. A crystalline polymorph of a given drug substance is chemically identical to any other crystalline polymorph of that drug substance in containing the same atoms bonded to one another in the same way, but differs in its crystal forms, which can affect one or more physical properties, such as stability, solubility, melting point, bulk density, flow properties, bioavailability, etc.

The term "composition" denotes one or more substance in a physical form, such as solid, liquid, gas, or a mixture thereof. One example of composition is a pharmaceutical composition, i.e., a composition related to, prepared for, or used in medical treatment.

The term "carboxylic acid" refers to an organic acid characterized by one or more carboxyl groups, such as acetic acid and oxalic acid. "Sulfonic acid" refers to an organic acid with the general formula of R-(S(O)₂-OH)ₙ, wherein R is an organic moiety and n is an integer above zero, such as 1, 2, and 3. The term "polyhydroxy acid" refers to a carboxylic acid containing two or more hydroxyl groups. Examples of polyhydroxy acid include, but are not limited to, lactobionic acid, gluconic acid, and galactose.

As used herein, "pharmaceutically acceptable" means suitable for use in contact with the tissues of humans and animals without undue toxicity, irritation, allergic response, and the like, commensurate with a reasonable benefit/risk ratio, and effective for their intended use within the scope of sound medical judgment.

"Salts" include derivatives of an active agent, wherein the active agent is modified by making acid or base addition salts thereof. Preferably, the salts are pharmaceutically acceptable salts. Such salts include, but are not limited to, pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Base addition salts include but are not limited to, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris-(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e. g., lysine and arginine dicyclohexylamine and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like. Standard methods for the preparation of pharmaceutically acceptable salts and their formulations are well known in the art, and are disclosed in various references, including for example, "Remington: The Science and Practice of Pharmacy", A. Gennaro, ed., 20th edition, Lippincott, Williams & Wilkins, Philadelphia, PA.

As used herein, "solvate" means a complex formed by solvation (the combination of solvent molecules with molecules or ions of the active agent of the present invention), or an aggregate that consists of a solute ion or molecule (the active agent of the present invention) with one or more solvent molecules. In the present invention, the preferred solvate is hydrate. Examples of hydrate include, but are not limited to, hemihydrate, monohydrate, dihydrate, trihydrate, hexahydrate, etc. It should be understood by one of ordinary skill in the art that the pharmaceutically acceptable salt of the present compound may also exist in a solvate form. The solvate is typically formed via hydration which is either part of the preparation of the present compound or through natural absorption of moisture by the anhydrous compound of the present invention. Solvates including hydrates may be consisting in stoichiometric ratios, for example, with two, three, four salt molecules per solvate or per hydrate molecule. Another possibility, for example, that two salt molecules are stoichiometric related to three, five, seven solvent or hydrate molecules. Solvents used for crystallization, such as alcohols, especially methanol and ethanol; aldehydes; ketones, especially acetone; esters, e.g. ethyl acetate; may be embedded in the crystal grating. Preferred are pharmaceutically acceptable solvents.

The term "substantially similar" as used herein means an analytical spectrum, such as XRPD patterns, Raman spectroscopy, and etc., which resembles the reference spectrum to a great degree in both the peak locations and their intensity.

The terms "excipient", "carrier", and "vehicle" are used interchangeably throughout this application and denote a substance with which a compound of the present invention is administered.

"Therapeutically effective amount" means the amount of a crystalline form that, when administered to a patient for treating a disease or other undesirable medical condition, is sufficient to have a beneficial effect with respect to that disease or condition. The therapeutically effective amount will vary depending on the crystalline form, the disease or condition and its severity, and the age, weight, etc. of the patient to be treated. Determining the therapeutically effective amount of a given crystalline form is within the ordinary skill of the art and requires no more than routine experimentation.

As used herein, the phrase "a disorder characterized by cell proliferation" or "a condition characterized by cell proliferation" include, but are not limited to, cancer, benign and malignant tumors. Examples of cancer and tumors include, but are not limited to, cancers or tumor growth of the colorectum, breast, lung, liver, pancreas, lymph node, colon, prostate, brain, head and neck, skin, kidney, blood and heart (e.g., leukemia, lymphoma, and carcinoma).

The terms "treat", "treating" or "treatment" in reference to a particular disease or disorder includes prevention of the disease or disorder, and/or lessening, improving, ameliorating or abrogating the symptoms and/or pathology of the disease or disorder. Generally the terms as used herein refer to ameliorating, alleviating, lessening, and removing symptoms of a disease or condition. A candidate molecule or compound described herein may be in a therapeutically effective amount in a formulation or medicament, which is an amount that can lead to a biological effect, such as apoptosis of certain cells (e.g., cancer cells), reduction of proliferation of certain cells, or lead to ameliorating, alleviating, lessening, or removing symptoms of a disease or condition, for example. The terms also can refer to reducing or stopping a cell proliferation rate (e.g., slowing or halting tumor growth) or reducing the number of proliferating cancer cells (e.g., removing part or all of a tumor). These terms also are applicable to reducing a titre of a microorganism in a system (i.e., cell, tissue, or subject) infected with a microorganism, reducing the rate of microbial propagation, reducing the number of symptoms or an effect of a symptom associated with the microbial infection, and/or removing detectable amounts of the microbe from the system. Examples of microorganism include but are not limited to virus, bacterium and fungus.

As used herein, the terms "inhibiting" or "reducing" cell proliferation is meant to slow down, to decrease, or, for example, to stop the amount of cell proliferation, as measured using methods known to those of ordinary skill in the art, by, for example, 10%, 20%, 30%, 40%, 50%, 60%, 70%, 80%, 90%, 95%, or 100%, when compared to proliferating cells that are not subjected to the methods and compositions of the present application.

As used herein, the term "apoptosis" refers to an intrinsic cell self-destruction or suicide program. In response to a triggering stimulus, cells undergo a cascade of events including cell shrinkage, blebbing of cell membranes and chromatic condensation and fragmentation. These events culminate in cell conversion to clusters of membrane-bound particles (apoptotic bodies), which are thereafter engulfed by macrophages.

### Crystalline Materials

In one embodiment, the present disclosure provides a crystalline form of Compound I (free base). In another embodiment, the present disclosure provides a crystalline form of a salt and/or solvate of Compound I. In one embodiment, the present invention provides a crystalline form of a salt of Compound I where the salt is a hydrochloric acid addition salt. In one example of the present disclosure, the salt is a sulfuric acid addition salt. In one example of the present disclosure, the salt is a sulfonic acid addition salt. In one example of the present disclosure, the salt is a carboxylic acid addition salt. In one example of the present disclosure, the salt is a polyhydroxy acid addition salt.

Examples of the crystalline salt include, but are not limited to, hydrochloric acid salt, maleic acid salt, fumaric acid salt, citric acid salt, malic acid salt, sulfuric acid salt, acetic acid salt, phosphoric acid salt, L-(+)-tartaric acid salt, D-glucuronic acid salt, benzoic acid salt, succinic acid salt, ethane sulfonic acid salt, methane sulfonic acid salt, p-toluene sulfonic acid salt, malonic acid salt, benzene sulfonic acid salt, and 1-hydroxy-2-naphthoic acid salt. In one example, the ratio of the Compound I to acid in the crystalline salt is about 1:0.5 to about 1:3.

An example of crystalline solvate includes, but not limited to NMP (N-methyl-2-pyrrolidone) solvate of Compound I.

In one example, the crystalline forms are characterized by the interlattice plane intervals determined by an X-ray powder diffraction pattern (XRPD). The spectrum of XRPD is typically represented by a diagram plotting the intensity of the peaks versus the location of the peaks, i.e., diffraction angle 2θ (two-theta) in degrees. The intensities are often given in parenthesis with the following abbreviations: very strong = vst; strong = st; medium = m; weak = w; and very weak = vw. The characteristic peaks of a given XRPD can be selected according to the peak locations and their relative intensity to conveniently distinguish this crystalline structure from others. The % intensity of the peaks relative to the most intense peak may be represented as I/Io.

Those skilled in the art recognize that the measurements of the XRPD peak locations and/or intensity for a given crystalline form of the same compound will vary within a margin of error. The values of degree 2θ allow appropriate error margins. Typically, the error margins are represented by "±". For example, the degree 2θ of about "8.716±0.3" denotes a range from about 8.716+0.3, i.e., about 9.016, to about 8.716-0.3, i.e., about 8.416. Depending on the sample preparation techniques, the calibration techniques applied to the instruments, human operational variation, and etc., those skilled in the art recognize that the appropriate error of margins for a XRPD can be about ±0.7; ±0.6; ±0.5; ±0.4; ±0.3; ±0.2; ±0.1; ±0.05; or less.

Additional details of the methods and equipment used for the XRPD analysis are described in the Examples section.

In one example, the crystalline forms are characterized by Differential Scanning Calorimetry (DSC). The DSC thermogram is typically expressed by a diagram plotting the normalized heat flow in units of Watts/gram ("W/g") versus the measured sample temperature in degree C. The DSC thermogram is usually evaluated for extrapolated onset and end (outset) temperatures, peak temperature, and heat of fusion. A peak characteristic value of a DSC thermogram is often used as the characteristic peak to distinguish this crystalline structure from others.

Those skilled in the art recognize that the measurements of the DSC thermogram for a given crystalline form of the same compound will vary within a margin of error. The values of a single peak characteristic value, expressed in degree C, allow appropriate error margins. Typically, the error margins are represented by "±". For example, the single peak characteristic value of about "53.09±2.0" denotes a range from about 53.09+2, i.e., about 55.09, to about 53.09-2, i.e., about 51.09. Depending on the sample preparation techniques, the calibration techniques applied to the instruments, human operational variations, and etc., those skilled in the art recognize that the appropriate error of margins for a single peak characteristic value can be ±2.5; ±2.0; ±1.5; ±1.0; ±0.5; or less.

Additional details of the methods and equipment used for the DSC thermogram analysis are described in the Examples section.

In one example, the crystalline forms are characterized by Raman spectroscopy. The Raman spectrum is typically represented by a diagram plotting the Raman intensity of the peaks versus the Raman shift of the peaks. The "peaks" of Raman spectroscopy are also known as "absorption bands". The intensities are often given in parenthesis with the following abbreviations: strong = st; medium = m; and weak = w. The characteristic peaks of a given Raman spectrum can be selected according to the peak locations and their relative intensity to conveniently distinguish this crystalline structure from others.

Those skilled in the art recognize that the measurements of the Raman peak shifts and/or intensity for a given crystalline form of the same compound will vary within a margin of error. The values of peak shift, expressed in reciprocal wave numbers (cm⁻¹), allow appropriate error margins. Typically, the error margins are represented by "±". For example, the Raman shift of about "1310±10" denotes a range from about 1310+10, i.e., about 1320, to about 1310-10, i.e., about 1300. Depending on the sample preparation techniques, the calibration techniques applied to the instruments, human operational variations, and etc., those skilled in the art recognize that the appropriate error of margins for a Raman shift can be ±12; ±10; ±8; ±5; ±3; ±1; or less.

Additional details of the methods and equipment used for the Raman spectroscopy analysis are described in the Examples section.

In one example, the crystalline form of Compound I (free base). In some examples, crystalline form of Compound I (free base) exhibits different polymorphs. Examples of the crystalline form of Compound I (free base) include, but are not limited to, Polymorphs A, C, E, and G, as defined in the following sections.

In some examples, one form of polymorph is more stable than the other forms. In one example, crystalline form of polymorph A of compound I (free base) exhibits high stability. In some examples, different polymorphs of Compound I (free base) convert to another polymorph forms under certain conditions. In some examples, polymorphs C, E, and/or G convert to polymorph A under suitable conditions. In one example, each of polymorphs C, E, and G independently convert to polymorph A in a solution at room temperature or at an elevated temperature. Polymorph A is the most thermodynamically stable form; however, other forms may be formed or favored kinetically under certain conditions.

In one example of the present disclosure, the crystalline form of Compound I may comprise of a mixture of one or more forms of polymorphs of Compound I. In some examples, the crystalline form of Compound I may comprise of substantially pure form of one polymorph type. In one example, the crystalline form of Compound I may comprise of over about 99.9%, about 99.8%, about 99.7%, about 99.6%, about 99.5%, about 99.4%, about 99.3%, about 99.2%, about 99.1%, or about 99.0% of one polymorph of Compound I. In another example, the crystalline form of Compound I may comprise over about 99%, 98%, 97%, 96%, 95%, 94%, 93%, 92%, 91%, or 90% of one polymorph of Compound I. In some examples, the crystalline form of Compound I may comprise over about 90%, 85%, 80%, 75%, 70%, 65%, 60%, 55%, 50%, 45%, or 40% of one polymorph of Compound I.

In one example of the present disclosure, the crystalline form of Compound I may comprise of at least about 99.9%, about 99.8%, about 99.7%, about 99.6%, about 99.5%, about 99.4%, about 99.3%, about 99.2%, about 99.1%, about 99.0 %, about 98%, about 97%, about 96%, about 95%, about 94%, about 93%, about 92%, about 91%, about 90%, or about 85% of polymorph A of Compound I (free base).

In one example of the present disclosure, the crystalline form of Compound I may be polymorph A of Compound I (free base) comprising about 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 18%, or 20% of polymorphs C, E, or G, or mixtures thereof.

In one example of the present disclosure, the crystalline form of Compound I may be polymorph C of Compound I (free base) comprising about 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 18%, or 20% of polymorph A.

In one example of the present disclosure, the crystalline form of Compound I may be polymorph E of Compound I (free base) comprising about 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 18%, or 20% of polymorph A.

In one example of the present disclosure, the crystalline form of Compound I may be polymorph G of Compound I (free base) comprising about 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 18%, or 20% of polymorph A.

In one example, the present disclosure provides crystalline form of Compound I that is highly pure. In one example of the present disclosure, the purity of the crystalline form of Compound I may be at least 99.9%, about 99.8%, about 99.7%, about 99.6%, about 99.5%, about 99.4%, about 99.3%, about 99.2%, about 99.1%, about 99.0 %, about 98%, about 97%, about 96%, about 95%, about 94%, about 93%, about 92%, about 91%, or about 90% pure with respect to Compound I.

Additional methods to characterize the present crystalline forms are described in the Example section of this application.

### Polymorph A

In one example, Polymorph A of Compound I (free base) exhibits an XRPD comprising peaks at about 7.730, 22.050, and 24.550 degrees two-theta with the margin of error of about ±0.5; about ±0.4; about ±0.3; about ±0.2; about ±0.1; about ±0.05; or less. In another example, the XRPD of the crystalline polymorph A of Compound I (free base) further comprises peaks at about 9.410 and 27.700 degrees two-theta with the margin of error of about ±0.5; about ±0.4; about ±0.3; about ±0.2; about ±0.1; about ±0.05; or less. In further example, the crystalline form of polymorph A of Compound I (free base) further comprises peaks at about 17.950 and 25.400 degrees two-theta with the margin of error of about±0.5; about ±0.4; about ±0.3; about ±0.2; about ±0.1; about ±0.05; or less. In another example, the crystalline form of polymorph A of Compound I (free base) further comprises at least one peaks at about 11.230, 11.630, 16.900, 18.580, 23.300, and 26.700 degrees two-theta with the margin of error of about±0.5; about ±0.4; about ±0.3; about ±0.2; about ±0.1; about ±0.05; or less. In yet another example, the crystalline form of polymorph A of Compound I (free base) exhibits an XRPD comprising peaks shown in Table 1 below:

**Table 1. XRPD Table of Polymorph A of Compound I (free base)**

| **2Theta** | **Flex Width** | **d-value** | **Intensity** | **I/Io** |
|---|---|---|---|---|
| 3.830 | ***** | 23.0507 | 312 | 7 |
| 7.730 | 0.200 | 11.4275 | 4749 | 100 |
| 9.410 | 0.224 | 9.3908 | 2036 | 43 |
| 11.230 | 0.200 | 7.8726 | 1322 | 28 |
| 11.630 | 0.224 | 7.6027 | 1254 | 27 |
| 14.060 | 0.224 | 6.2937 | 485 | 11 |
| 16.900 | 0.224 | 5.2419 | 1246 | 27 |
| 17.190 | 0.141 | 5.1541 | 507 | 11 |
| 17.950 | 0.224 | 4.9376 | 1599 | 34 |
| 18.580 | 0.235 | 4.7716 | 1338 | 29 |
| 19.700 | 0.259 | 4.5027 | 875 | 19 |
| 20.210 | 0.129 | 4.3903 | 467 | 10 |
| 20.360 | 0.176 | 4.3582 | 528 | 12 |
| 20.940 | 0.247 | 4.2388 | 1115 | 24 |
| 21.510 | 0.188 | 4.1278 | 816 | 18 |
| 22.050 | 0.247 | 4.0279 | 2994 | 64 |
| 23.300 | 0.235 | 3.8145 | 1285 | 28 |
| 23.690 | 0.224 | 3.7526 | 714 | 16 |
| 24.550 | 0.235 | 3.6231 | 2447 | 52 |
| 25.400 | 0.212 | 3.5037 | 1804 | 38 |
| 26.180 | 0.212 | 3.4011 | 646 | 14 |
| 26.700 | 0.235 | 3.3360 | 1330 | 28 |
| 27.700 | 0.235 | 3.2178 | 2224 | 47 |
| 28.300 | 0.212 | 3.1509 | 531 | 12 |
| 28.540 | 0.200 | 3.1250 | 533 | 12 |
| 30.700 | 0.247 | 2.9099 | 436 | 10 |
| 31.230 | 0.282 | 2.8617 | 518 | 11 |
| 31.560 | 0.176 | 2.8325 | 436 | 10 |
| 36.040 | 0.247 | 2.4900 | 670 | 15 |
| 38.680 | 0.353 | 2.3259 | 422 | 9 |

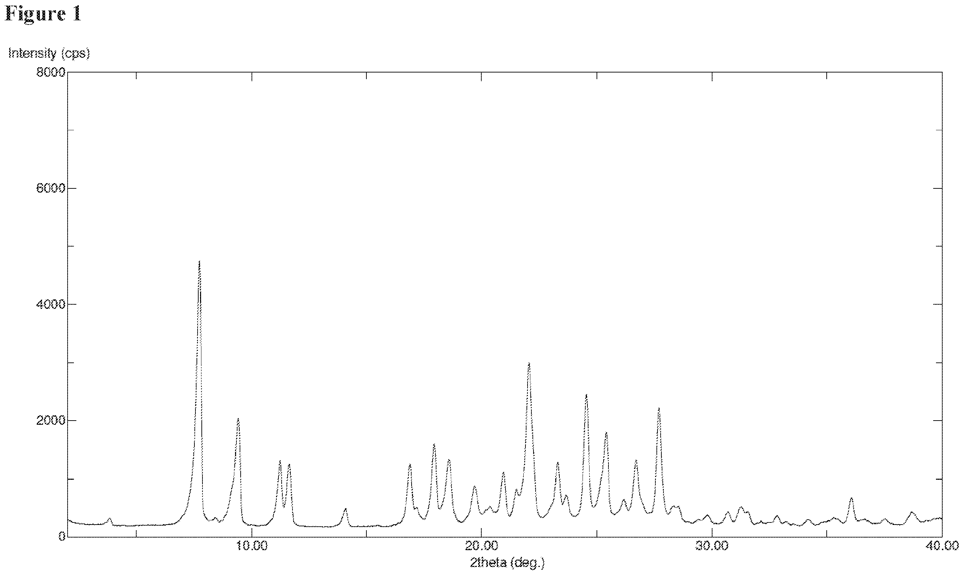

In one specific example, the crystalline form of polymorph A of Compound I (free base) exhibits an XRPD that is substantially similar to Figure 1.

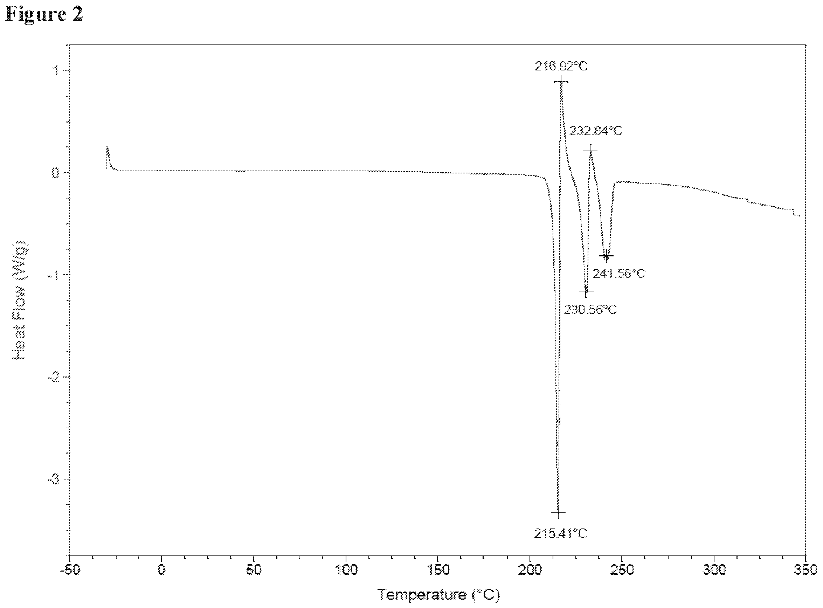

In one example, the crystalline form of polymorph A of Compound I (free base) exhibits a DSC thermogram comprising a sharp endotherm at about 215.41 °C with the error of margin of about ±2.5; about ±2.0; about ±1.5; about ±1.0; about ±0.5; or less. In another example, the crystalline form of polymorph A of Compound I (free base) further exhibits a DSC thermogram comprising one or more of the following peaks: exotherm at about 216.92±2.0 °C, endotherm at about 230.56±2.0 °C, exotherm at about 232.84±2.0 °C, and endotherm at about 241.56±2.0 °C. In one specific example, the crystalline form of polymorph A of Compound I (free base) exhibits a DSC thermogram that is substantially similar to Figure 2.

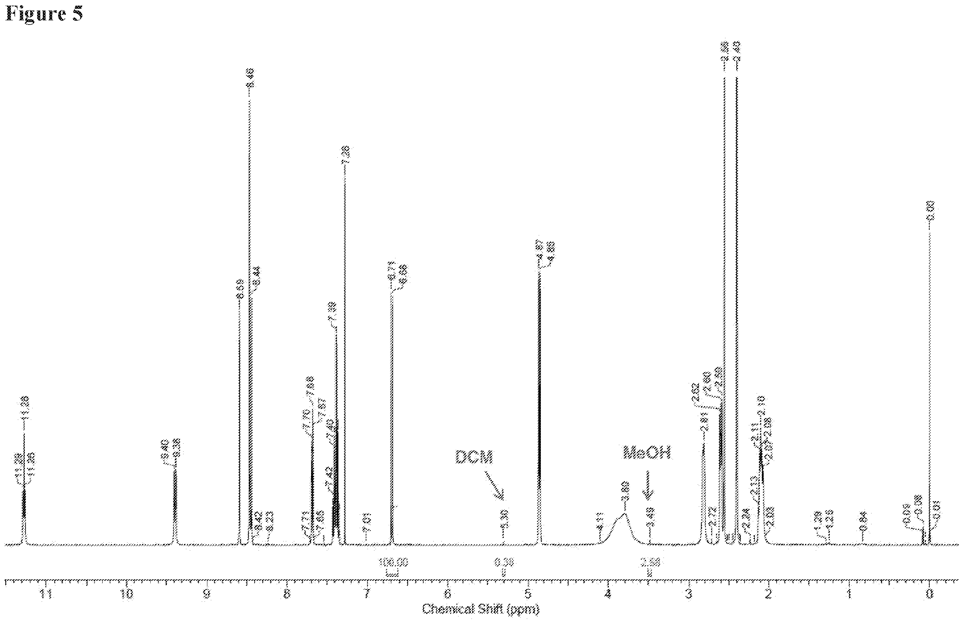

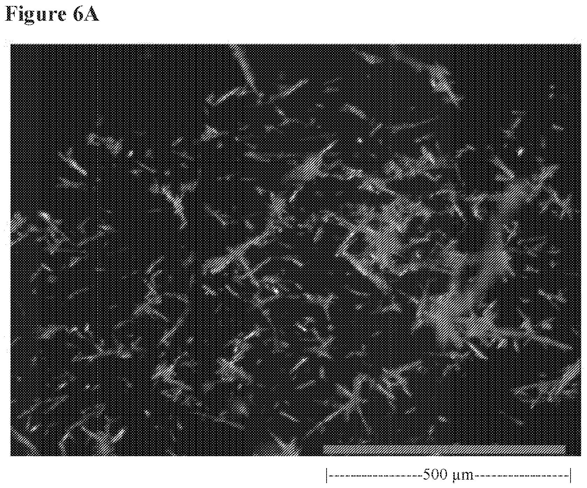

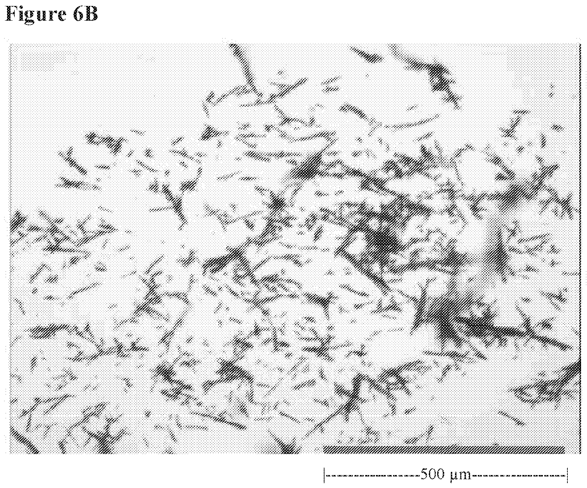

In one example, the crystalline form of polymorph A of Compound I (free base) exhibits a ¹H NMR spectrum that is substantially similar to Figure 5 In another example, the crystalline form of polymorph A of Compound I (free base) may have the crystal habit of thin needles that form loosely bound lumps on a macroscopic scale, which are substantially similar to Figures 6A and 6B.

### Polymorph C

In one example, polymorph C of Compound I (free base) exhibits an XRPD comprising peaks at about 5.720 degrees two-theta with the margin of error of about ±0.5; about ±0.4; about ±0.3; about ±0.2; about ±0.1; about ±0.05; or less. In another example, the crystalline form of polymorph C of Compound I (free base) exhibits an XRPD comprising peaks shown in Table 2 below:

**Table 2. XRPD Table of Polymorph C of Compound I (free base)**

| **2Theta** | **Flex Width** | **d-value** | **Intensity** | **I/Io** |
|---|---|---|---|---|
| 4.700 | 0.259 | 18.7856 | 1578 | 8 |
| 5.720 | 0.282 | 15.4378 | 21302 | 100 |
| 7.900 | 0.306 | 11.1820 | 355 | 2 |
| 10.940 | 0.282 | 8.0806 | 597 | 3 |
| 11.360 | 0.306 | 7.7828 | 852 | 4 |
| 12.480 | 0.212 | 7.0867 | 1205 | 6 |
| 12.640 | 0.306 | 6.9974 | 1422 | 7 |
| 15.700 | 0.400 | 5.6398 | 803 | 4 |
| 17.420 | 0.329 | 5.0866 | 778 | 4 |
| 17.940 | 0.329 | 4.9403 | 770 | 4 |
| 22.220 | 0.329 | 3.9974 | 364 | 2 |
| 23.540 | 0.376 | 3.7762 | 808 | 4 |
| 24.860 | 0.259 | 3.5786 | 448 | 3 |
| 25.940 | 0.329 | 3.4320 | 866 | 5 |
| 27.080 | 0.353 | 3.2901 | 597 | 3 |
| 29.200 | 0.282 | 3.0558 | 793 | 4 |

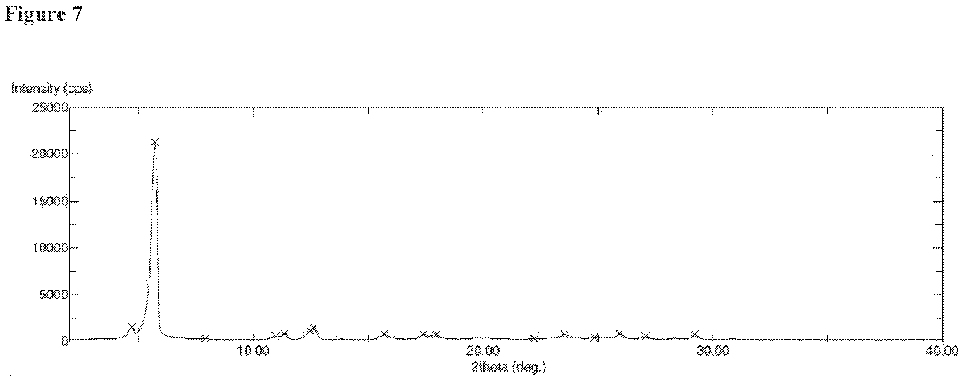

In one specific example, the crystalline form of polymorph C of Compound I (free base) exhibits an XRPD that is substantially similar to Figure 7.

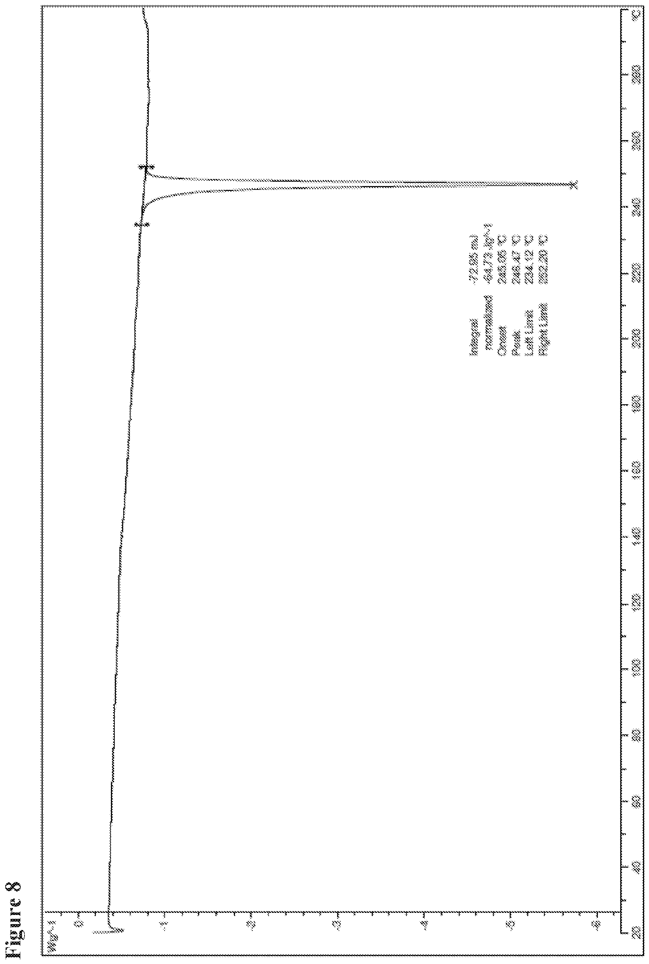

In one example, the crystalline form of polymorph C of Compound I (free base) exhibits a DSC thermogram comprising a peak characteristic value at about 246.47 °C with the error of margin of about ±2.5; about ±2.0; about ±1.5; about ±1.0; about ±0.5; or less. In one specific example, the crystalline form of polymorph C of Compound I (free base) exhibits a DSC thermogram that is substantially similar to Figure 8.

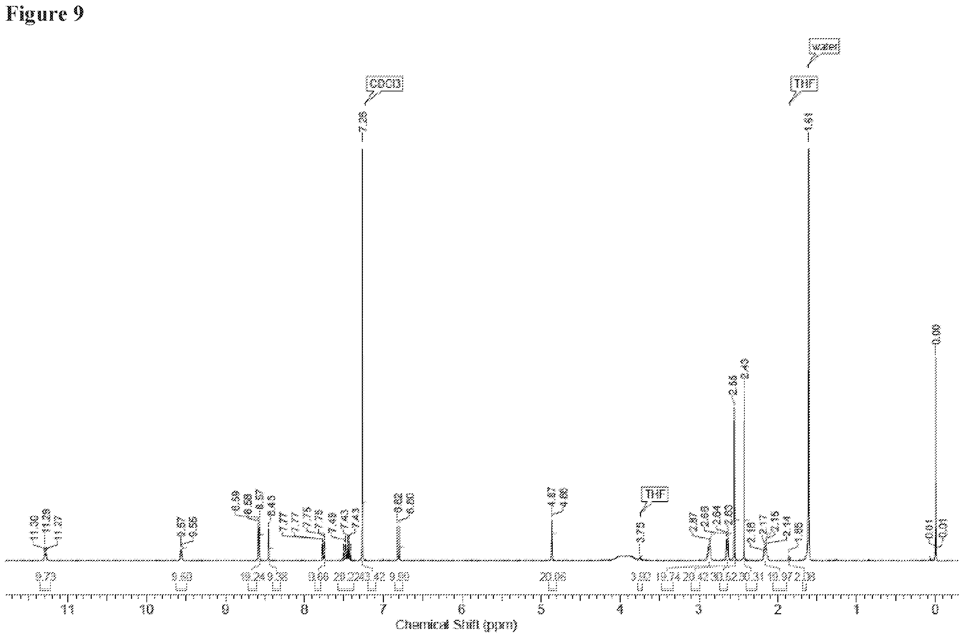

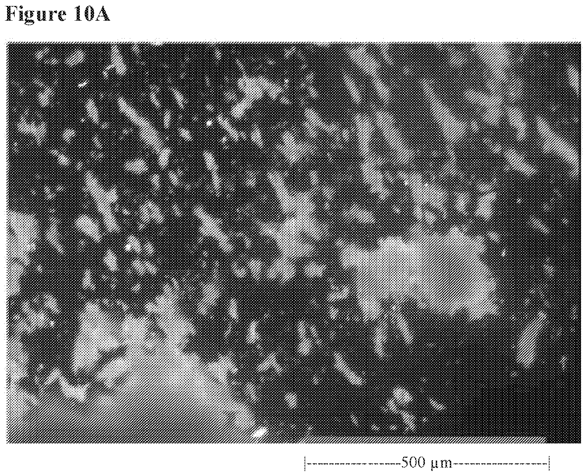

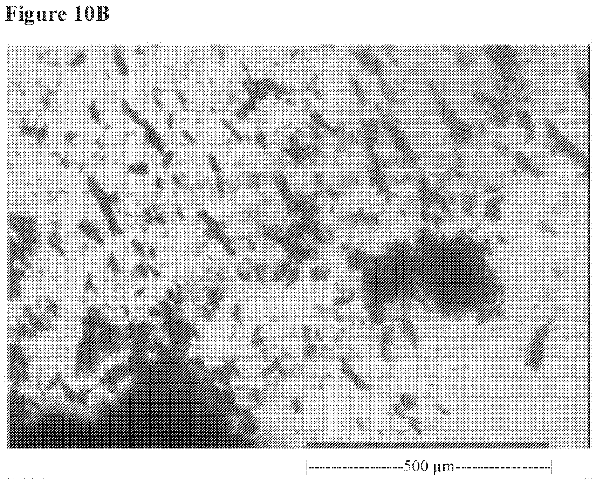

In one example, the crystalline form of polymorph C of Compound I (free base) exhibits a ¹H NMR spectrum that is substantially similar to Figure 9. In another example, the crystalline form of polymorph C of Compound I (free base) may consist of agglomerates of needles on a macroscopic scale, which are substantially similar to Figures 10A and 10B.

### Polymorph E

In one example, Polymorph E of Compound I (free base) exhibits an XRPD comprising peaks at about 5.680 degrees two-theta with the margin of error of about ±0.5; about ±0.4; about ±0.3; about ±0.2; about ±0.1; about ±0.05; or less. In another example, the XRPD of the crystalline polymorph E of Compound I (free base) further comprises at least one peak at about 12.200, 12.600, 25.360, and 27.560 degrees two-theta with the margin of error of about ±0.5; about ±0.4; about ±0.3; about ±0.2; about ±0.1; about ±0.05; or less. In yet another example, the crystalline form of polymorph E of Compound I (free base) exhibits an XRPD comprising peaks shown in the Table 3 below:

**Table 3. XRPD Table of Polymorph E of Compound I (free base)**

| **2Theta** | **Flex Width** | **d-value** | **Intensity** | **I/Io** |
|---|---|---|---|---|
| 5.680 | 0.188 | 15.5465 | 22340 | 100 |
| 7.700 | 0.188 | 11.4720 | 450 | 3 |
| 10.020 | 0.188 | 8.8204 | 427 | 2 |
| 11.340 | 0.282 | 7.7965 | 1248 | 6 |
| 11.720 | 0.165 | 7.5445 | 562 | 3 |
| 12.200 | 0.188 | 7.2487 | 1655 | 8 |
| 12.600 | 0.212 | 7.0195 | 2116 | 10 |
| 13.300 | 0.188 | 6.6516 | 417 | 2 |
| 13.660 | 0.188 | 6.4771 | 358 | 2 |
| 14.820 | 0.188 | 5.9726 | 834 | 4 |
| 15.040 | 0.141 | 5.8857 | 523 | 3 |
| 15.500 | 0.282 | 5.7121 | 401 | 2 |
| 16.220 | 0.282 | 5.4601 | 1410 | 7 |
| 16.720 | 0.165 | 5.2979 | 458 | 3 |
| 17.060 | 0.212 | 5.1931 | 506 | 3 |
| 18.000 | 0.188 | 4.9240 | 773 | 4 |
| 18.460 | 0.212 | 4.8023 | 575 | 3 |
| 19.080 | 0.235 | 4.6476 | 350 | 2 |
| 20.280 | 0.400 | 4.3753 | 538 | 3 |
| 20.680 | 0.235 | 4.2915 | 1016 | 5 |
| 21.060 | 0.212 | 4.2149 | 493 | 3 |
| 21.460 | 0.165 | 4.1373 | 476 | 3 |
| 21.980 | 0.259 | 4.0405 | 462 | 3 |
| 22.560 | 0.188 | 3.9380 | 917 | 5 |
| 22.940 | 0.212 | 3.8736 | 1385 | 7 |
| 23.680 | 0.188 | 3.7542 | 838 | 4 |
| 23.980 | 0.188 | 3.7079 | 1432 | 7 |
| 24.500 | 0.235 | 3.6304 | 472 | 3 |
| 25.360 | 0.212 | 3.5092 | 1579 | 8 |
| 27.560 | 0.212 | 3.2338 | 1975 | 9 |
| 28.120 | 0.353 | 3.1707 | 500 | 3 |
| 28.680 | 0.212 | 3.1100 | 1112 | 5 |
| 29.360 | 0.235 | 3.0395 | 393 | 2 |
| 30.000 | 0.282 | 2.9761 | 476 | 3 |
| 30.700 | 0.165 | 2.9099 | 552 | 3 |
| 30.980 | 0.165 | 2.8842 | 455 | 3 |
| 31.840 | 0.259 | 2.8082 | 492 | 3 |
| 39.800 | ^{∗∗∗∗∗} | 2.2630 | 402 | 2 |

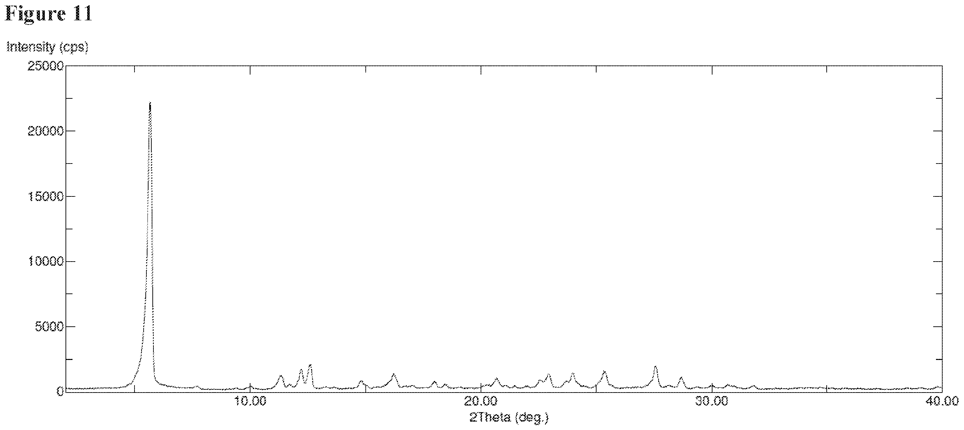

In one specific example, the crystalline form of polymorph E of Compound I (free base) exhibits an XRPD that is substantially similar to Figure 11.

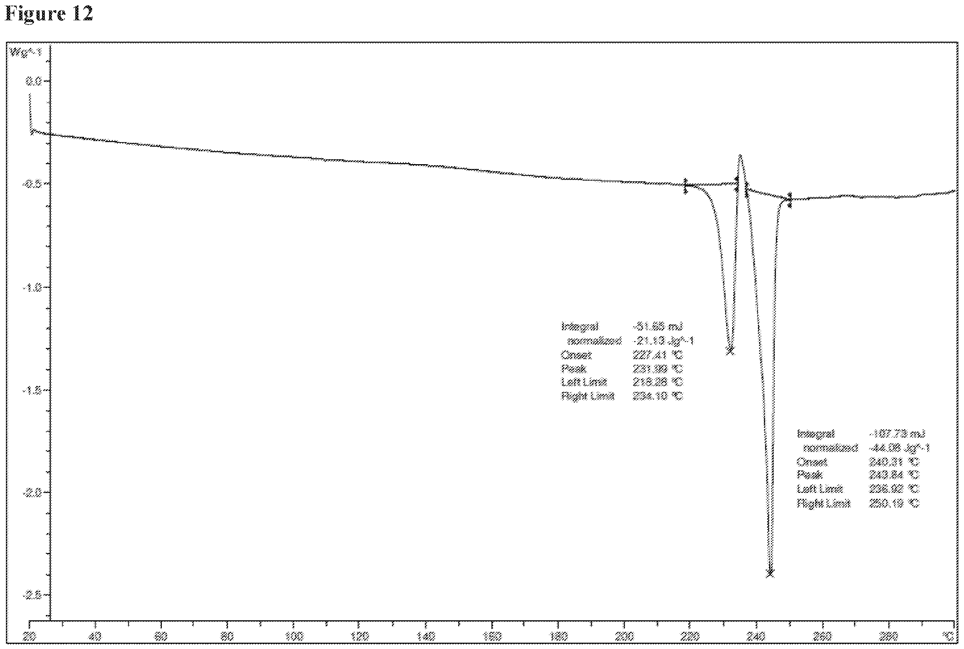

In one example, the crystalline form of polymorph E of Compound I (free base) exhibits a DSC thermogram comprising a peak characteristic value at about 231.99 °C with the error of margin of about ±2.5; about ±2.0; about ±1.5; about ±1.0; about ±0.5; or less. In one specific example, the crystalline form of polymorph E of Compound I (free base) exhibits a DSC thermogram that is substantially similar to Figure 12.

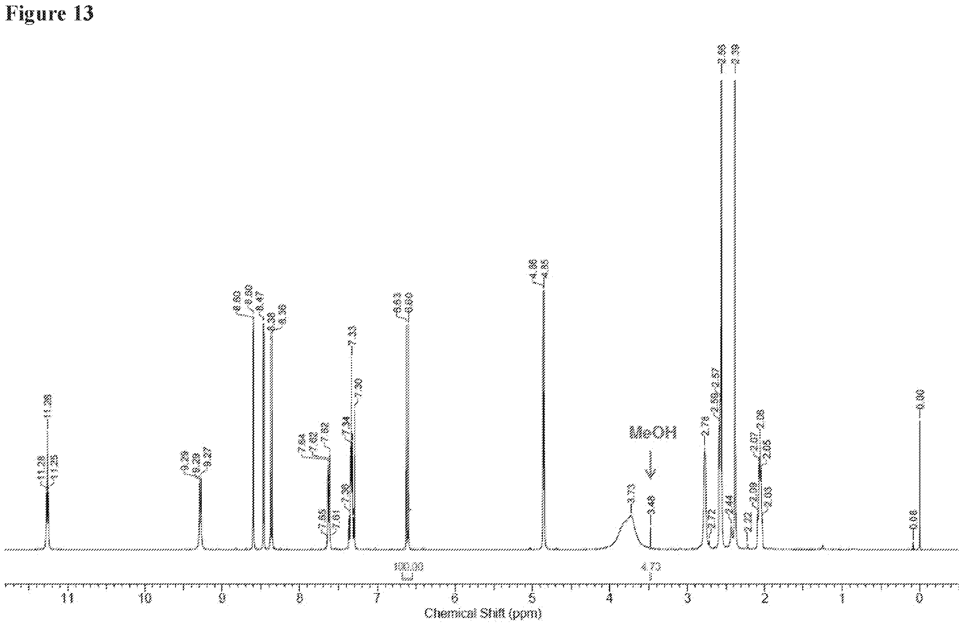

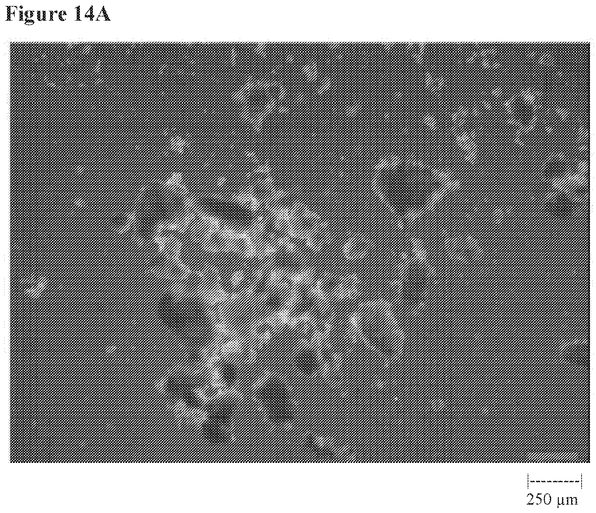

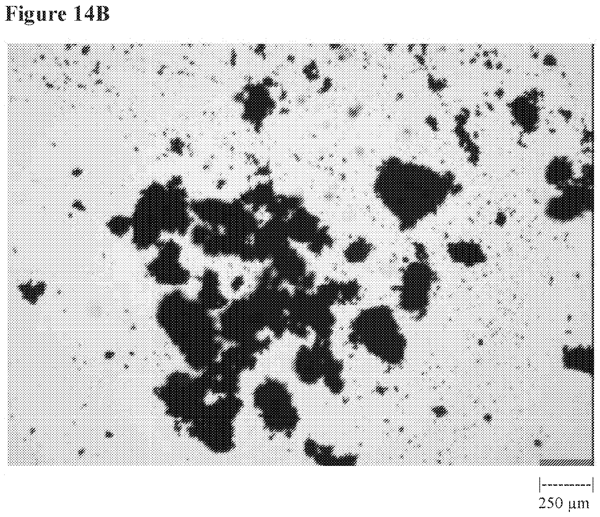

In one example, the crystalline form of polymorph E of Compound I (free base) exhibits a ¹H NMR spectrum that is substantially similar to Figure 13. In another example, the crystalline form of polymorph E of Compound I (free base) may be substantially similar to Figures 14A and 14B.

### Polymorph G

In one example, polymorph G of Compound I (free base) exhibits an XRPD comprising peaks at about 5.000 and 6.060 degrees two-theta with the margin of error of about ±0.5; about ±0.4; about ±0.3; about ±0.2; about ±0.1; about ±0.05; or less. In another example, the crystalline form of polymorph G of Compound I (free base) exhibits an XRPD comprising peaks shown in Table 4 below:

**Table 4. XRPD Table of Polymorph G of Compound I (free base)**

| **2Theta** | **Flex Width** | **d-value** | **Intensity** | **I/Io** |
|---|---|---|---|---|
| 5.000 | 0.212 | 17.6591 | 3953 | 32 |
| 6.060 | 0.306 | 14.5724 | 12680 | 100 |
| 7.800 | 0.282 | 11.3251 | 485 | 4 |
| 9.760 | 0.259 | 9.0548 | 503 | 4 |
| 10.360 | 0.282 | 8.5317 | 412 | 4 |
| 13.400 | 0.259 | 6.6022 | 868 | 7 |
| 15.000 | 0.329 | 5.9014 | 625 | 5 |
| 15.980 | 0.541 | 5.5416 | 709 | 6 |
| 18.120 | 0.212 | 4.8916 | 393 | 4 |
| 19.120 | 0.282 | 4.6380 | 390 | 4 |
| 22.320 | 0.306 | 3.9798 | 480 | 4 |
| 23.480 | 0.259 | 3.7857 | 1035 | 9 |
| 26.000 | 0.259 | 3.4242 | 729 | 6 |
| 26.400 | 0.259 | 3.3732 | 865 | 7 |
| 27.700 | 0.282 | 3.2178 | 505 | 4 |

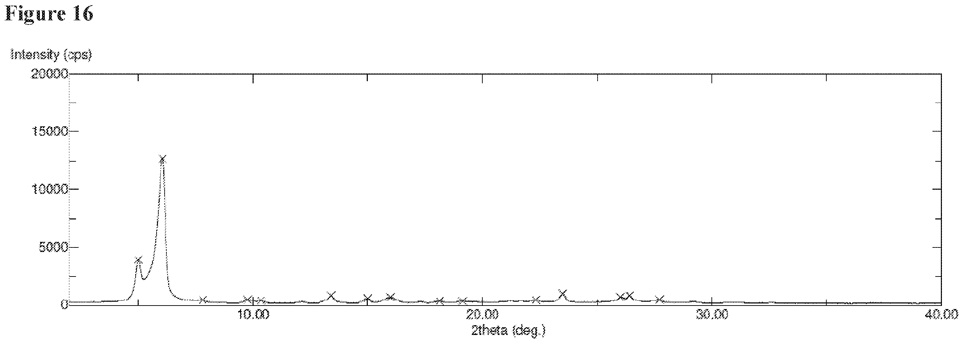

In one specific example, the crystalline form of polymorph G of Compound I (free base) exhibits an XRPD that is substantially similar to Figure 16.

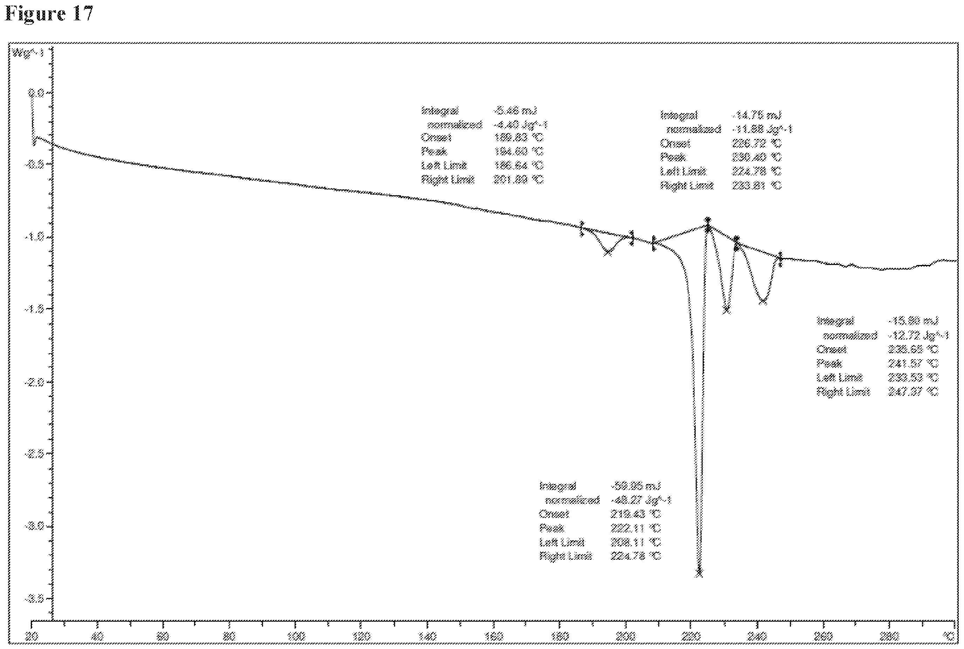

In one example, the crystalline form of polymorph G of Compound I (free base) exhibits a DSC thermogram comprising a peak characteristic value at about 222.11 °C with the error of margin of about ±2.5; about ±2.0; about ±1.5; about ±1.0; about ±0.5; or less. In one specific example, the crystalline form of polymorph G of Compound I (free base) exhibits a DSC thermogram that is substantially similar to Figure 17.

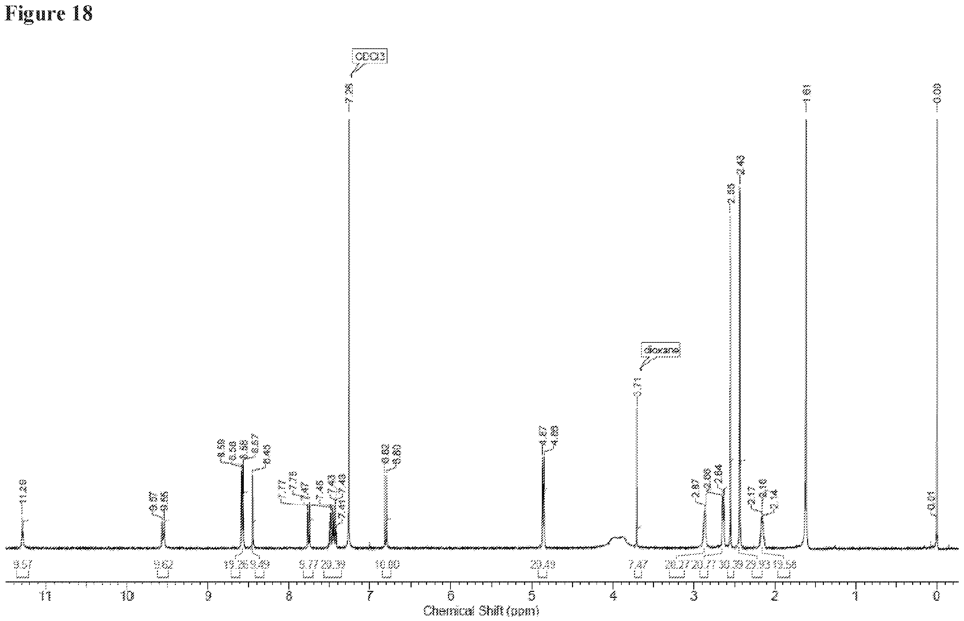

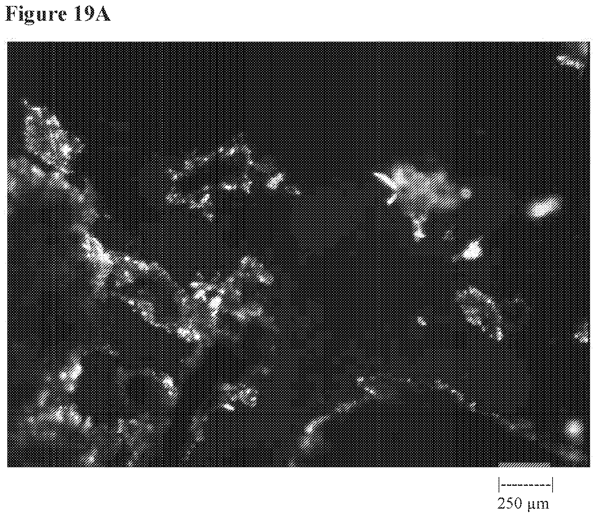

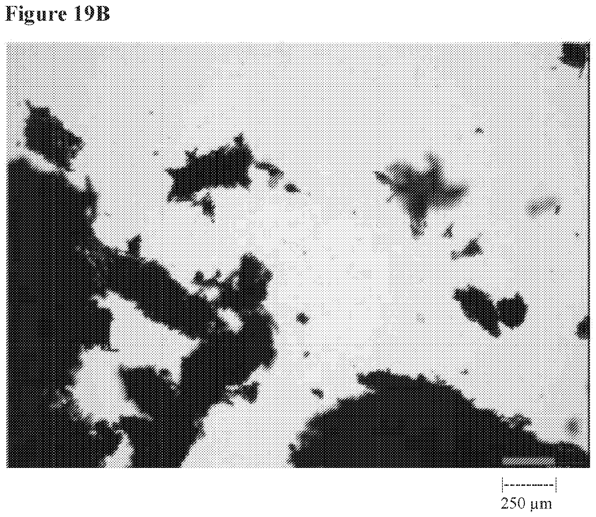

In one example, the crystalline form of polymorph G of Compound I (free base) exhibits a ¹H NMR spectrum that is substantially similar to Figure 18. In another example, the crystalline form of polymorph G of Compound I (free base) may be substantially similar to Figures 19A and 19B.

### HCl Salt of Compound I

In one example, the crystalline form of HCl salt of Compound I exhibits an XRPD comprising peaks at about 4.660 and 24.540 degrees two-theta with the margin of error of about ±0.5; about ±0.4; about ±0.3; about ±0.2; about ±0.1; about ±0.05; or less. In another example, the XRPD of the crystalline form of HCl salt of Compound I further comprises one, two, three, or four peaks selected from at about 19.260, 20.160, 24.920, and 26.360 degrees two-theta with the margin of error of about ±0.5; about ±0.4; about ±0.3; about ±0.2; about ±0.1; about ±0.05; or less. In another example, the XRPD of the crystalline form of HCl salt of Compound I further comprises one, two, or three peaks selected from at about 13.980, 14.540, 25.380 and 28.940 degrees two-theta with the margin of error of about ±0.5; about ±0.4; about ±0.3; about ±0.2; about ±0.1; about ±0.05; or less. In yet another example, the crystalline form of a HCl salt of Compound I exhibits an XRPD comprising peaks shown in the table below:

**Table 6. XRPD Table of a HCl Salt of Compound I**

| **2Theta** | **Flex Width** | **d-value** | **Intensity** | **I/Io** |
|---|---|---|---|---|
| 4.660 | 0.212 | 18.9468 | 4530 | 100 |
| 9.680 | 0.259 | 9.1294 | 622 | 14 |
| 13.980 | 0.306 | 6.3295 | 792 | 18 |
| 14.540 | 0.259 | 6.0870 | 790 | 18 |
| 15.720 | 0.306 | 5.6326 | 534 | 12 |
| 15.980 | 0.235 | 5.5416 | 600 | 14 |
| 16.740 | 0.400 | 5.2917 | 491 | 11 |
| 17.340 | 0.306 | 5.1099 | 460 | 11 |
| 18.580 | 0.353 | 4.7716 | 507 | 12 |
| 19.260 | 0.306 | 4.6046 | 895 | 20 |
| 20.160 | 0.329 | 4.4010 | 874 | 20 |
| 20.680 | 0.212 | 4.2915 | 418 | 10 |
| 22.160 | 0.212 | 4.0081 | 437 | 10 |
| 22.900 | 0.259 | 3.8803 | 523 | 12 |
| 23.360 | 0.188 | 3.8049 | 484 | 11 |
| 23.680 | 0.188 | 3.7542 | 446 | 10 |
| 24.540 | 0.282 | 3.6245 | 2963 | 66 |
| 24.920 | 0.212 | 3.5701 | 1581 | 35 |
| 25.380 | 0.259 | 3.5064 | 777 | 18 |
| 26.360 | 0.471 | 3.3783 | 998 | 23 |
| 26.940 | 0.212 | 3.3068 | 662 | 15 |
| 28.160 | 0.259 | 3.1663 | 580 | 13 |
| 28.500 | 0.188 | 3.1293 | 671 | 15 |
| 28.940 | 0.259 | 3.0827 | 797 | 18 |
| 29.380 | 0.188 | 3.0375 | 589 | 13 |
| 29.620 | 0.259 | 3.0134 | 554 | 13 |
| 30.440 | 0.188 | 2.9341 | 420 | 10 |

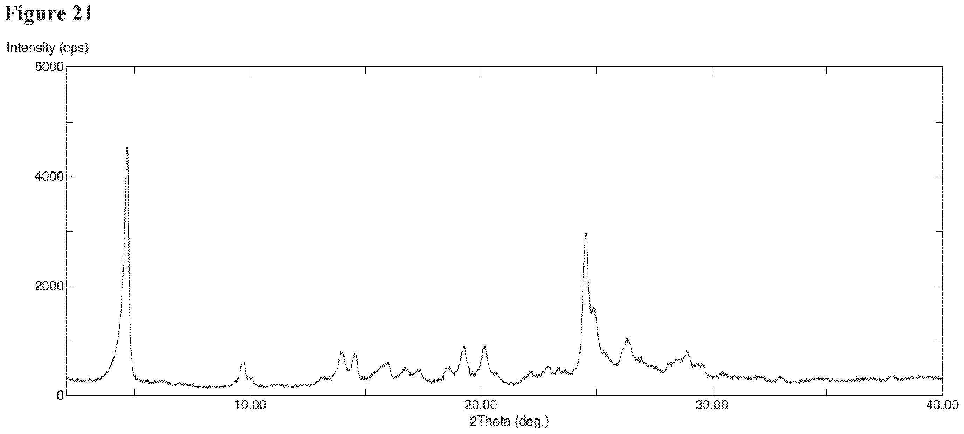

In one specific example, the crystalline form of a HCl salt of Compound I exhibits an XRPD that is substantially similar to Figure 21.

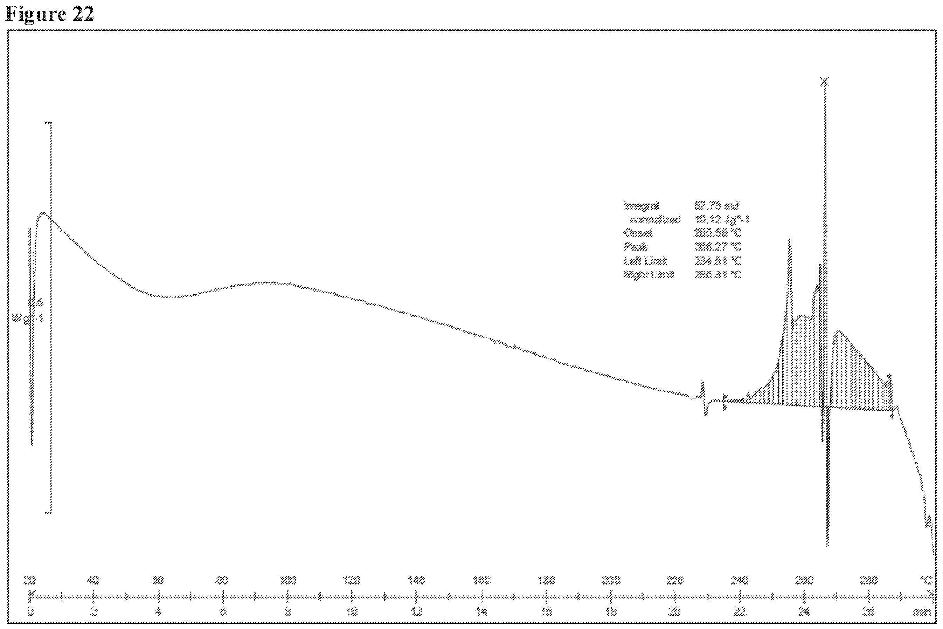

In one example, the crystalline form of a HCl salt of Compound I exhibits a DSC thermogram comprising a peak characteristic value at about 266.27 °C with the error of margin of about ±2.5; about ±2.0; about ±1.5; about ±1.0; about ±0.5; or less. In one specific example, the crystalline form of a HCl salt of Compound I exhibits a DSC thermogram that is substantially similar to Figure 22. In one example, there is no clear melting point for the crystalline form of a HCl salt of Compound I.

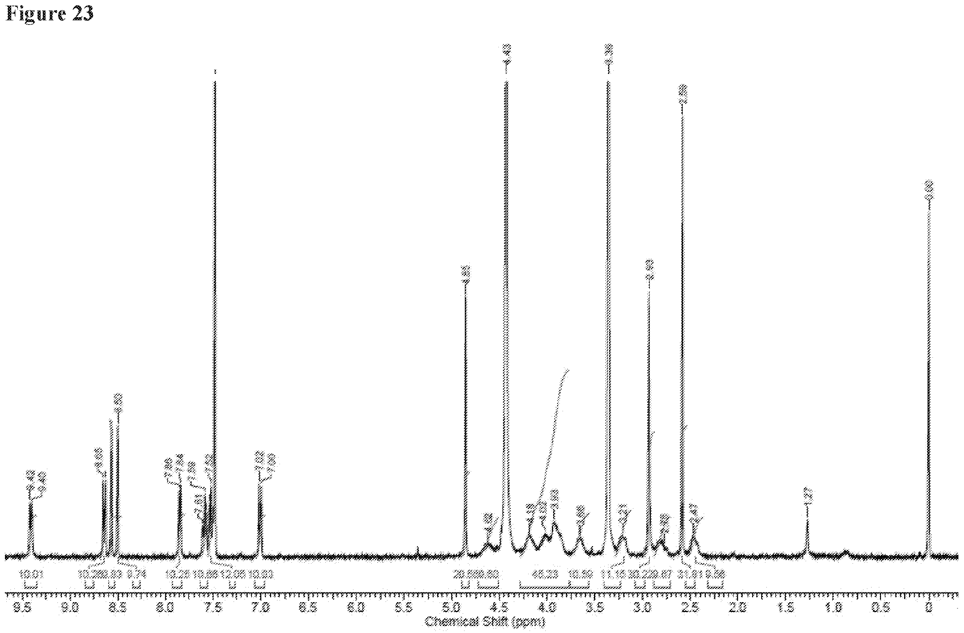

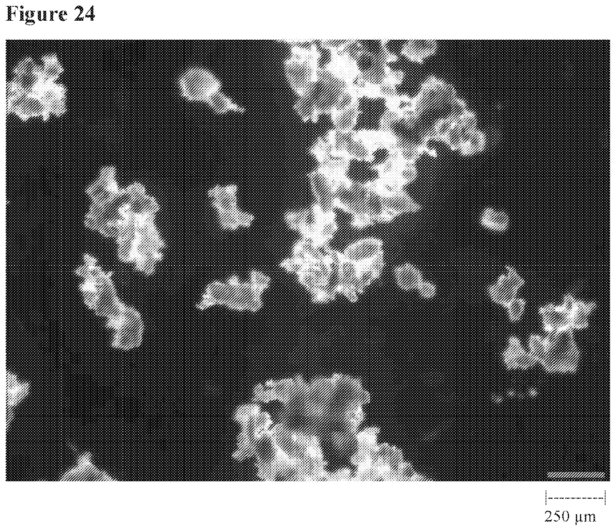

In one example, the crystalline form of a HCl salt of Compound I exhibits a ¹H NMR spectrum that is substantially similar to Figure 23. In another example, the crystalline form of a HCl salt of Compound I may consist of small needles that form agglomerates on a macroscopic scale, which may be substantially similar to Figures 24. In one example, the ratio of HCl and Compound I in the HCl salt of Compound I is about 1:1.

### Maleic Acid Salt of Compound I

In one example, the crystalline form of a maleic acid salt of Compound I exhibits an XRPD comprising peaks at about 7.400, 18.440, and 26.500 degrees two-theta with the margin of error of about ±0.5; about ±0.4; about ±0.3; about ±0.2; about ±0.1; about ±0.05; or less. In another example, the XRPD of the crystalline maleic acid salt of Compound I further comprises one, two, three, or four peaks selected from at about 22.320, 23.920, 24.300, and 25.240 degrees two-theta with the margin of error of about ±0.5; about ±0.4; about ±0.3; about ±0.2; about ±0.1; about ±0.05; or less. In another example, the XRPD of the crystalline maleic acid salt of Compound I further comprises one, two, three, or four peaks selected from at about 5.040, 15.080, 15.880, 20.860, and 28.540 degrees two-theta with the margin of error of about ±0.5; about ±0.4; about ±0.3; about ±0.2; about ±0.1; about ±0.05; or less. In yet another example, the crystalline form of a maleic acid salt of Compound I exhibits an XRPD comprising peaks shown in the table below:

**Table 7. XRPD Table of a Maleic Acid Salt of Compound I**

| **2Theta** | **Flex Width** | **d-value** | **Intensity** | **I/Io** |
|---|---|---|---|---|
| 5.040 | 0.235 | 17.5191 | 1648 | 48 |
| 6.280 | 0.212 | 14.0624 | 393 | 12 |
| 7.400 | 0.212 | 11.9364 | 3113 | 90 |
| 7.900 | 0.212 | 11.1820 | 424 | 13 |
| 8.480 | ***** | 10.4184 | 357 | 11 |
| 10.120 | 0.329 | 8.7335 | 435 | 13 |
| 10.400 | 0.188 | 8.4989 | 407 | 12 |
| 11.560 | 0.212 | 7.6486 | 577 | 17 |
| 12.180 | 0.329 | 7.2606 | 1336 | 39 |
| 13.100 | 0.282 | 6.7527 | 967 | 28 |
| 13.640 | 0.259 | 6.4865 | 1094 | 32 |
| 15.080 | 0.259 | 5.8702 | 1433 | 42 |
| 15.440 | 0.165 | 5.7342 | 707 | 21 |
| 15.880 | 0.306 | 5.5763 | 1600 | 46 |
| 17.220 | 0.259 | 5.1452 | 957 | 28 |
| 18.440 | 0.494 | 4.8075 | 2650 | 76 |
| 19.520 | 0.212 | 4.5439 | 691 | 20 |
| 19.800 | 0.235 | 4.4802 | 875 | 26 |
| 20.860 | 0.353 | 4.2549 | 1512 | 44 |
| 21.280 | 0.235 | 4.1719 | 946 | 28 |
| 22.320 | 0.329 | 3.9798 | 2242 | 65 |
| 23.580 | 0.165 | 3.7699 | 839 | 25 |
| 23.920 | 0.282 | 3.7171 | 1719 | 50 |
| 24.300 | 0.306 | 3.6598 | 2156 | 62 |
| 25.240 | 0.612 | 3.5256 | 1719 | 50 |
| 26.500 | 0.306 | 3.3607 | 3491 | 100 |
| 27.200 | 0.259 | 3.2758 | 681 | 20 |
| 27.880 | 0.306 | 3.1974 | 676 | 20 |
| 28.540 | 0.259 | 3.1250 | 1552 | 45 |
| 28.900 | 0.235 | 3.0869 | 1337 | 39 |
| 29.800 | 0.235 | 2.9957 | 757 | 22 |
| 30.220 | 0.165 | 2.9550 | 574 | 17 |
| 30.900 | 0.188 | 2.8915 | 565 | 17 |
| 31.180 | 0.165 | 2.8661 | 614 | 18 |
| 31.760 | 0.165 | 2.8151 | 612 | 18 |
| 33.560 | 0.282 | 2.6681 | 629 | 19 |
| 33.980 | 0.165 | 2.6361 | 531 | 16 |
| 34.300 | 0.259 | 2.6122 | 594 | 17 |
| 36.020 | 0.518 | 2.4913 | 608 | 18 |
| 37.360 | 0.306 | 2.4050 | 594 | 18 |

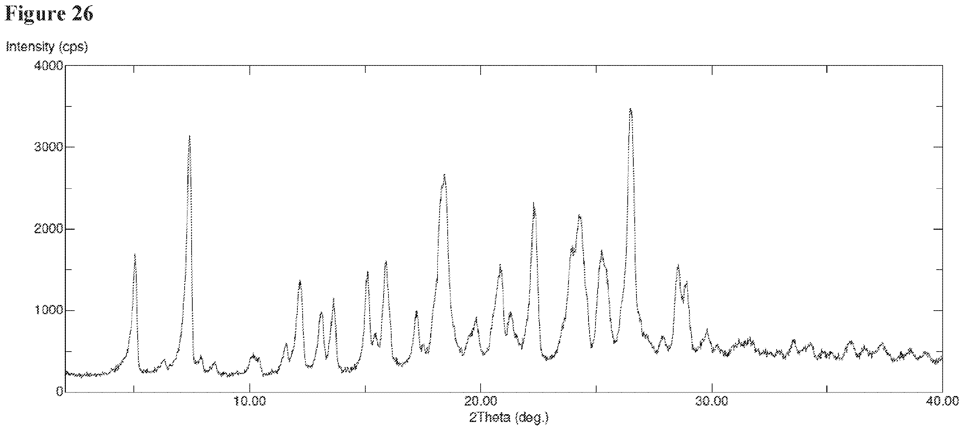

In one specific example, the crystalline form of a maleic acid salt of Compound I exhibits an XRPD that is substantially similar to Figure 26.

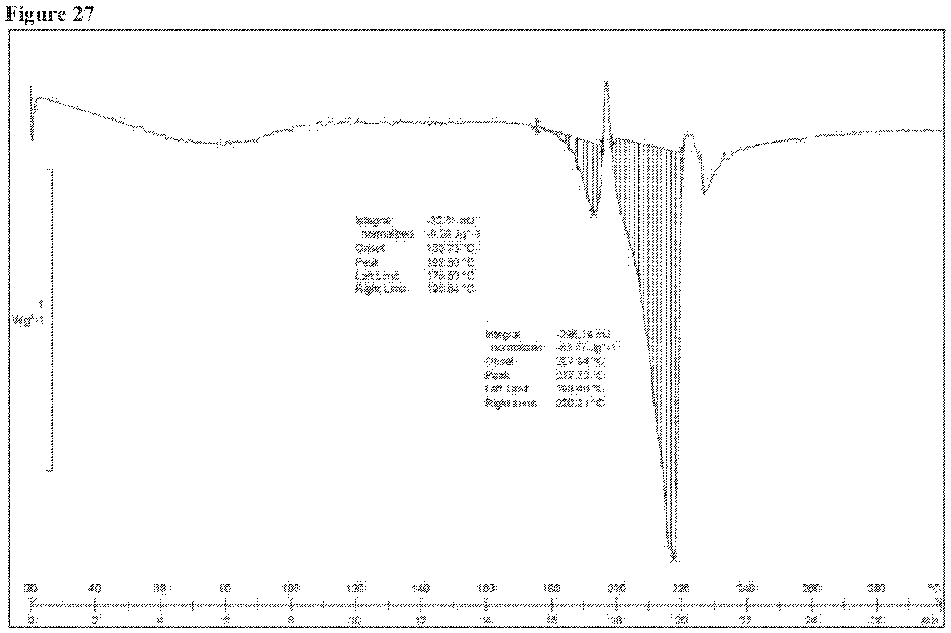

In one example, the crystalline form of maleic acid salt of Compound I exhibits a DSC thermogram comprising a peak characteristic value at about 217.32 °C with the error of margin of about ±2.5; about ±2.0; about ±1.5; about ±1.0; about ±0.5; or less. In one specific example, the crystalline form of a maleic acid salt of Compound I exhibits a DSC thermogram that is substantially similar to Figure 27.

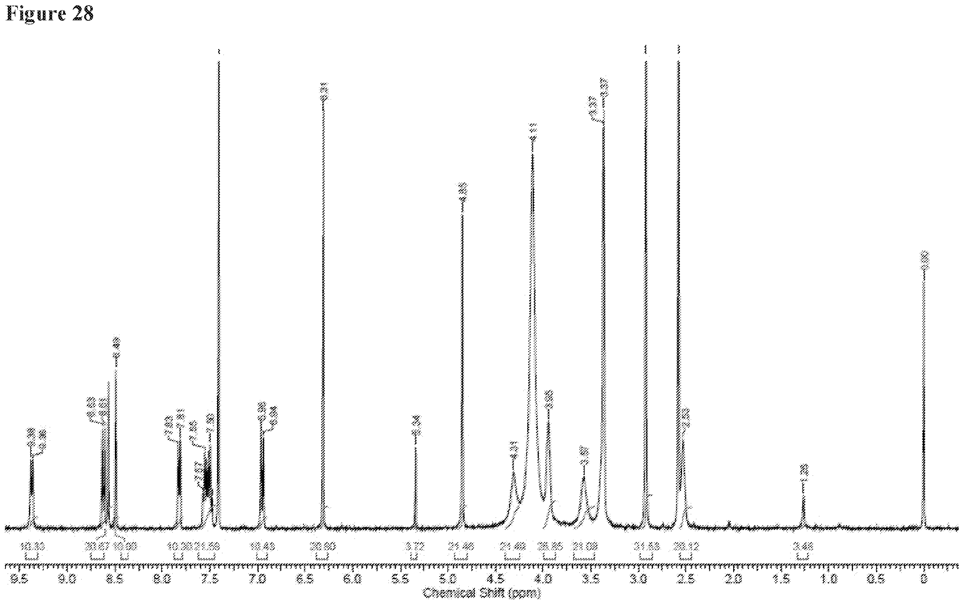

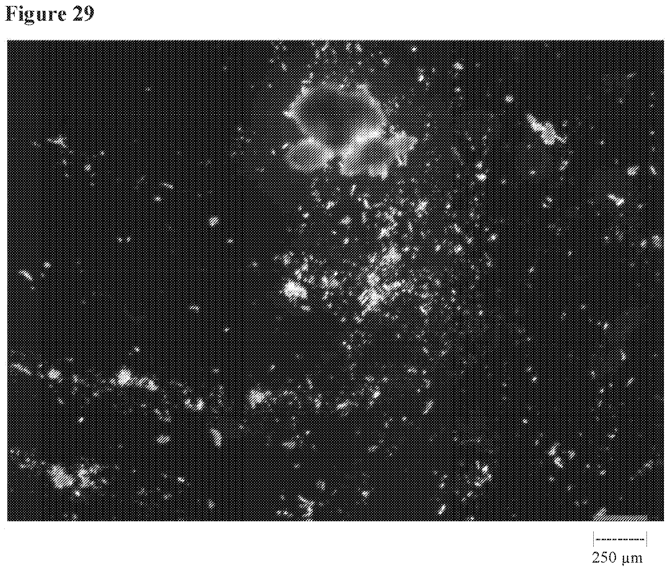

In one example, the crystalline form of a maleic acid salt of Compound I exhibits a ¹H NMR spectrum that is substantially similar to Figure 28. In another example, the crystalline form of a maleic salt of Compound I may consist of small needles that form agglomerates on a macroscopic scale, which may be substantially similar to Figures 29. In one example, the ratio of maleic acid and Compound I in the maleic acid salt of Compound I is about 1: 1.

### Fumaric Acid Salt of Compound I

In one example, the crystalline form of a fumaric acid salt of Compound I exhibits an XRPD comprising peaks at about 6.360 and 24.800 degrees two-theta with the margin of error of about ±0.5; about ±0.4; about ±0.3; about ±0.2; about ±0.1; about ±0.05; or less. In another example, the XRPD of the crystalline fumaric acid salt of Compound I further comprises at least one, two, or three peaks selected from at about 19.660, 20.420, and 26.860 degrees two-theta with the margin of error of about ±0.5; about ±0.4; about ±0.3; about ±0.2; about ±0.1; about ±0.05; or less. In another example, the XRPD of the crystalline fumaric acid salt of Compound I further comprises at least one, two, three, or four peaks at about 12.680, 17.020, 25.180, and 28.280 degrees two-theta with the margin of error of about ±0.5; about ±0.4; about ±0.3; about ±0.2; about ±0.1; about ±0.05; or less. In yet another example, the crystalline form of a fumaric acid salt of Compound I exhibits an XRPD comprising peaks shown in the table below:

**Table 8. XRPD Table of a Fumaric Acid Salt of Compound I**

| **2Theta** | **Flex Width** | **d-value** | **Intensity** | **I/Io** |
|---|---|---|---|---|
| 6.360 | 0.212 | 13.8857 | 3416 | 100 |
| 8.500 | 0.212 | 10.3940 | 599 | 18 |
| 10.860 | 0.212 | 8.1400 | 985 | 29 |
| 12.680 | 0.235 | 6.9754 | 1095 | 33 |
| 15.500 | 0.282 | 5.7121 | 822 | 25 |
| 16.360 | 0.212 | 5.4137 | 763 | 23 |
| 17.020 | 0.188 | 5.2052 | 1050 | 31 |
| 17.620 | 0.165 | 5.0293 | 451 | 14 |
| 18.040 | 0.259 | 4.9132 | 882 | 26 |
| 18.480 | 0.235 | 4.7972 | 611 | 18 |
| 19.660 | 0.235 | 4.5118 | 1654 | 49 |
| 20.420 | 0.259 | 4.3456 | 1917 | 57 |
| 21.260 | 0.212 | 4.1757 | 405 | 12 |
| 21.740 | 0.212 | 4.0846 | 519 | 16 |
| 22.020 | 0.165 | 4.0333 | 425 | 13 |
| 22.480 | 0.329 | 3.9518 | 455 | 14 |
| 23.100 | 0.541 | 3.8471 | 907 | 27 |
| 23.880 | 0.235 | 3.7232 | 711 | 21 |
| 24.180 | 0.212 | 3.6777 | 740 | 22 |
| 24.800 | 0.235 | 3.5871 | 2792 | 82 |
| 25.180 | 0.165 | 3.5338 | 1122 | 33 |
| 25.800 | 0.447 | 3.4503 | 938 | 28 |
| 26.300 | 0.259 | 3.3858 | 881 | 26 |
| 26.860 | 0.235 | 3.3165 | 1494 | 44 |
| 27.340 | 0.235 | 3.2594 | 905 | 27 |
| 27.900 | 0.188 | 3.1952 | 865 | 26 |
| 28.280 | 0.259 | 3.1531 | 1112 | 33 |
| 28.920 | 0.212 | 3.0848 | 720 | 22 |
| 30.200 | 0.282 | 2.9569 | 724 | 22 |

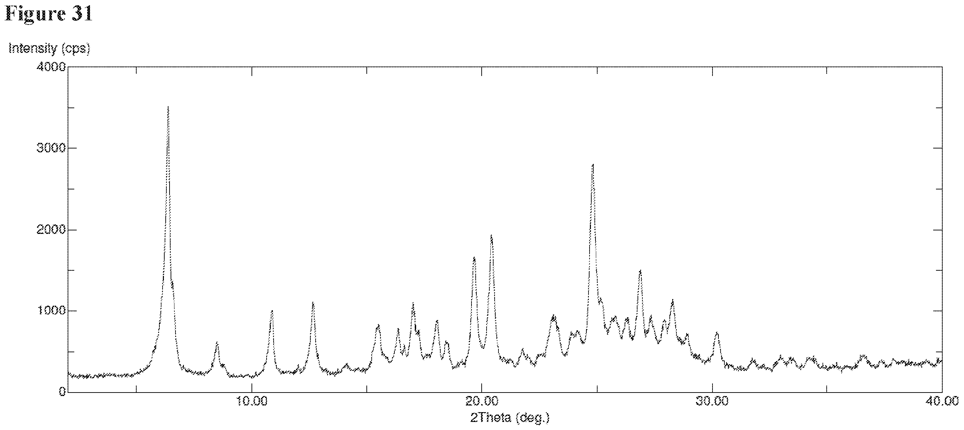

In one specific example, the crystalline form of a fumaric acid salt of Compound I exhibits an XRPD that is substantially similar to Figure 31.

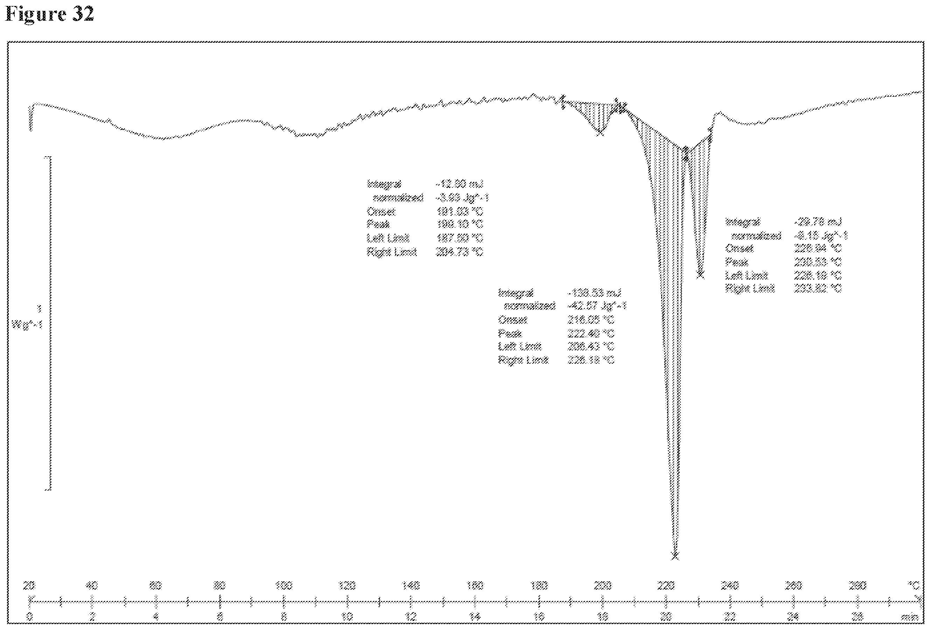

In one example, the crystalline form of a fumaric acid salt of Compound I exhibits a DSC thermogram comprising a peak characteristic value at about 222.40 °C with the error of margin of about ±2.5; about ±2.0; about ±1.5; about ±1.0; about ±0.5; or less. In one specific example, the crystalline form of a fumaric acid salt of Compound I exhibits a DSC thermogram that is substantially similar to Figure 32.

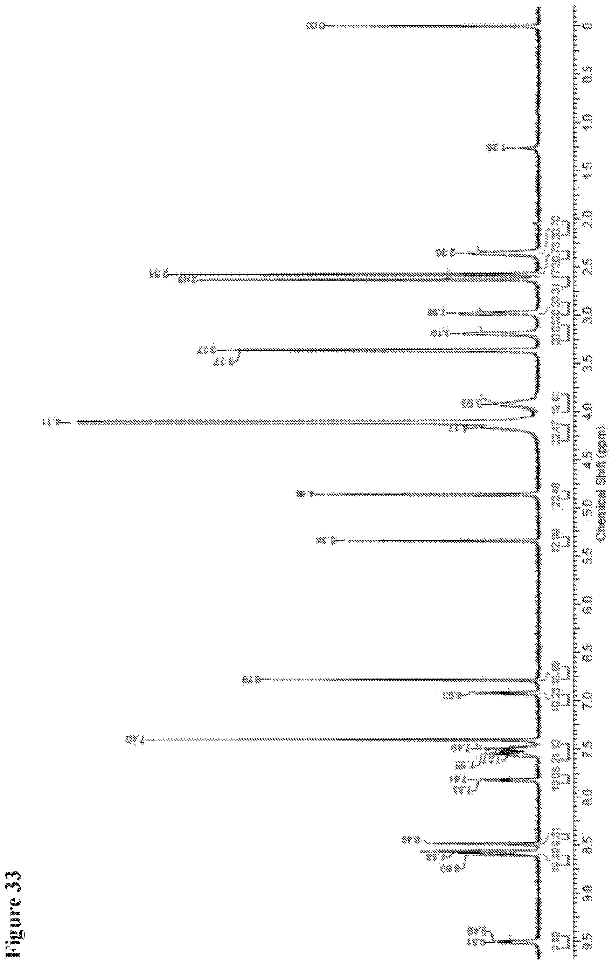

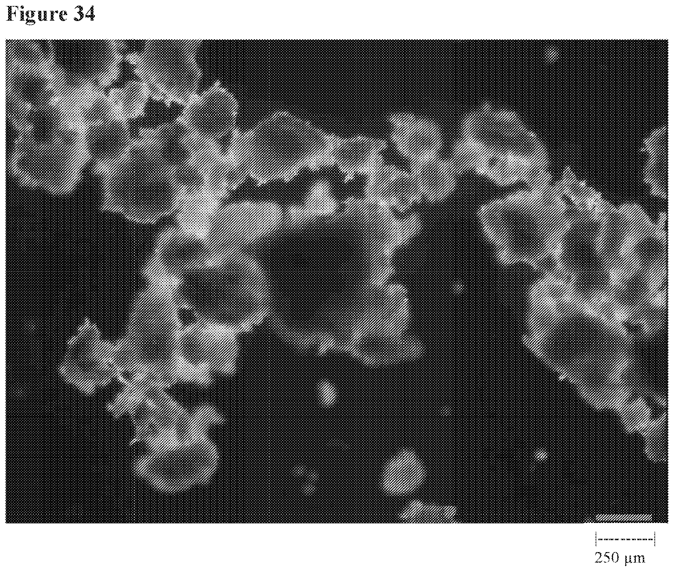

In one example, the crystalline form of a fumaric acid salt of Compound I exhibits a ¹H NMR spectrum that is substantially similar to Figure 33. In another example, the crystalline form of a fumaric acid salt of Compound I may consist of small needles that form agglomerates on a macroscopic scale, which may be substantially similar to Figures 34. In one example, the ratio of fumaric acid and Compound I in the fumaric acid salt of Compound I is about 1:1.

### Citric Acid Salt of Compound I

In one example, the crystalline form of a citric acid salt of Compound I exhibits an XRPD comprising peaks at about 4.900, 25.380, and 27.500 degrees two-theta with the margin of error of about ±0.5; about ±0.4; about ±0.3; about ±0.2; about ±0.1; about ±0.05; or less. In another example, the XRPD of the crystalline citric acid salt of Compound I further comprises at least one, two, three, or four peaks at about 15.360, 18.100, 19.300, and 26.140 degrees two-theta with the margin of error of about ±0.5; about ±0.4; about ±0.3; about ±0.2; about ±0.1; about ±0.05; or less. In another example, the XRPD of the crystalline citric acid salt of Compound I further comprises at least one, two, three, or four peaks at about 17.400, 18.680, 24.040, and 26.740 degrees two-theta with the margin of error of about ±0.5; about ±0.4; about ±0.3; about ±0.2; about ±0.1; about ±0.05; or less. In yet another example, the crystalline form of a citric acid salt of Compound I exhibits an XRPD comprising peaks shown in the table below:

**Table 9. XRPD Table of a Citric Acid Salt of Compound I**

| **2Theta** | **Flex Width** | **d-value** | **Intensity** | **I/Io** |
|---|---|---|---|---|
| 4.900 | 0.235 | 18.0193 | 1520 | 68 |
| 5.860 | 0.518 | 15.0693 | 606 | 28 |
| 9.560 | 0.212 | 9.2437 | 438 | 20 |
| 11.080 | 0.235 | 7.9788 | 898 | 41 |
| 12.820 | 0.235 | 6.8995 | 446 | 20 |
| 13.280 | 0.306 | 6.6616 | 506 | 23 |
| 14.480 | 0.188 | 6.1121 | 440 | 20 |
| 15.360 | 0.282 | 5.7638 | 1215 | 55 |
| 15.920 | 0.329 | 5.5623 | 585 | 27 |
| 16.200 | 0.165 | 5.4668 | 509 | 23 |
| 17.400 | 0.259 | 5.0924 | 1088 | 49 |
| 18.100 | 0.259 | 4.8970 | 1158 | 52 |
| 18.680 | 0.353 | 4.7462 | 984 | 44 |
| 19.300 | 0.282 | 4.5952 | 1209 | 55 |
| 19.700 | 0.235 | 4.5027 | 728 | 33 |
| 20.120 | 0.188 | 4.4097 | 740 | 34 |
| 20.380 | 0.306 | 4.3540 | 796 | 36 |
| 20.960 | 0.306 | 4.2348 | 649 | 30 |
| 21.380 | 0.212 | 4.1526 | 724 | 33 |
| 21.700 | 0.212 | 4.0920 | 654 | 30 |
| 22.120 | 0.212 | 4.0153 | 577 | 26 |
| 22.520 | 0.188 | 3.9449 | 607 | 28 |
| 23.140 | 0.259 | 3.8406 | 796 | 36 |
| 23.360 | 0.165 | 3.8049 | 706 | 32 |
| 24.040 | 0.306 | 3.6988 | 1055 | 48 |
| 24.580 | 0.282 | 3.6187 | 781 | 35 |
| 25.380 | 0.259 | 3.5064 | 2237 | 100 |
| 26.140 | 0.376 | 3.4062 | 1137 | 51 |
| 26.740 | 0.282 | 3.3311 | 1024 | 46 |
| 27.500 | 0.306 | 3.2408 | 2149 | 97 |
| 28.040 | 0.212 | 3.1796 | 620 | 28 |
| 28.540 | 0.188 | 3.1250 | 573 | 26 |
| 28.920 | 0.235 | 3.0848 | 616 | 28 |
| 30.280 | 0.471 | 2.9493 | 634 | 29 |

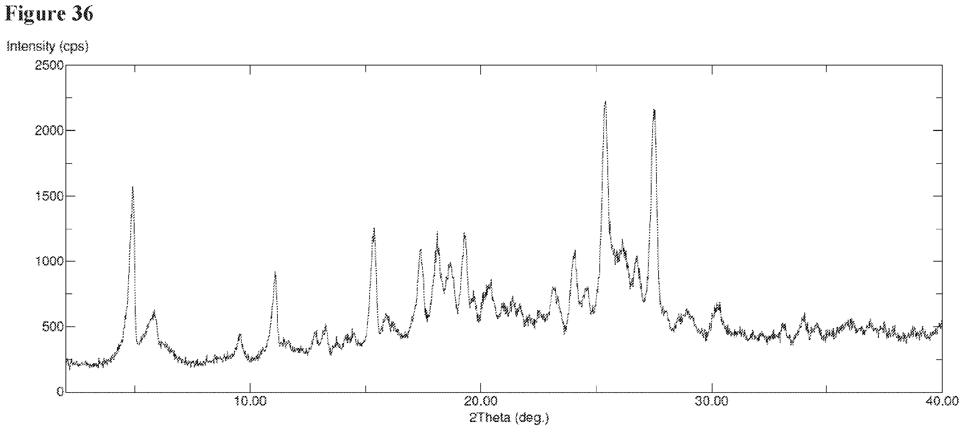

In one specific example, the crystalline form of a citric acid salt of Compound I exhibits an XRPD that is substantially similar to Figure 36.

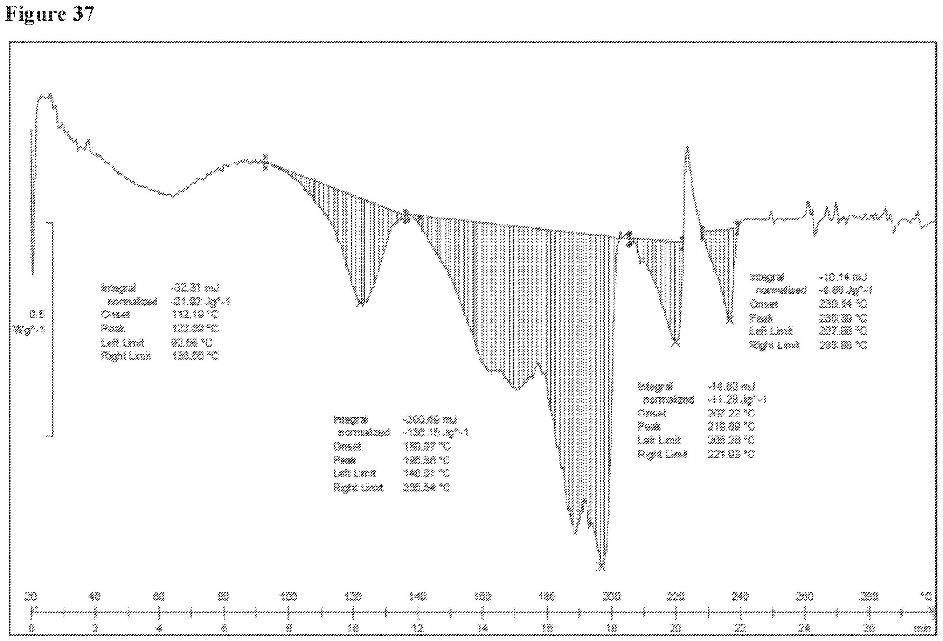

In one example, the crystalline form of a citric acid salt of Compound I exhibits a DSC thermogram comprising a peak characteristic value at about 196.86 °C with the error of margin of about ±2.5; about ±2.0; about ±1.5; about ±1.0; about ±0.5; or less. In one specific example, the crystalline form of a citric acid salt of Compound I exhibits a DSC thermogram that is substantially similar to Figure 37.

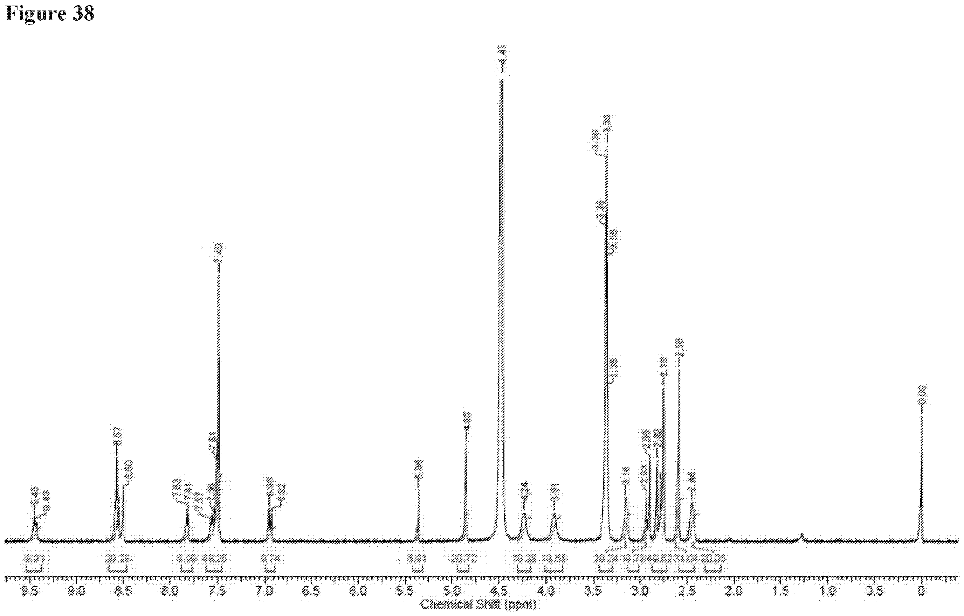

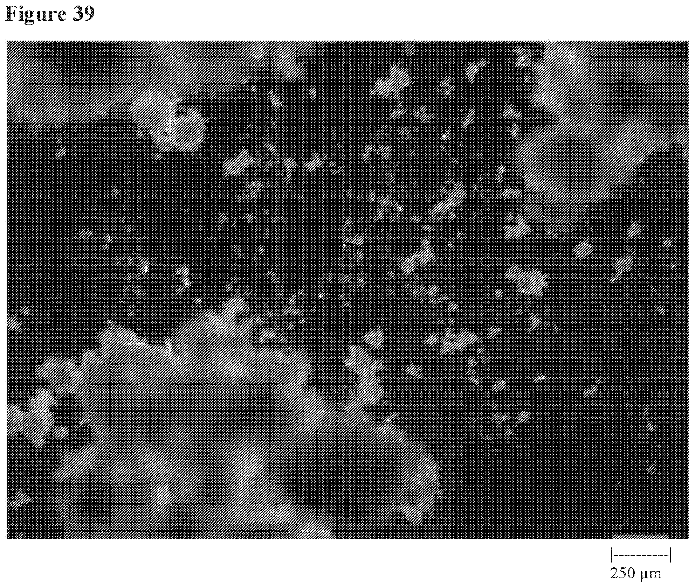

In one example, the crystalline form of a citric acid salt of Compound I exhibits a ¹H NMR spectrum that is substantially similar to Figure 38. In another example, the crystalline form of a citric acid salt of Compound I may consist of small needles that form agglomerates on a macroscopic scale, which may be substantially similar to Figures 39. In one example, the ratio of citric acid and Compound I in the citric acid salt of Compound I is about 1: 1.

### L-Malic Acid Salt of Compound I

In one example, the crystalline form of an L-malic acid salt of Compound I exhibits an XRPD comprising peaks at about 6.580, 6.780, and 25.560 degrees two-theta with the margin of error of about ±0.5; about ±0.4; about ±0.3; about ±0.2; about ±0.1; about ±0.05; or less. In another example, the XRPD of the crystalline L-malic acid salt of Compound I further comprises one, two, three, or four peaks selected from at about 19.560, 23.660, 26.060, and 26.960 degrees two-theta with the margin of error of about ±0.7; about ±0.6; about ±0.5; about ±0.4; about ±0.3; about ±0.2; about ±0.1; about ±0.05; or less. In another example, the XRPD of the crystalline L-malic acid salt of Compound I further comprises one, two, three, four or five peaks selected from at about 8.800, 11.800, 18.600, 24.460, and 25.080 degrees two-theta with the margin of error of about ±0.5; about ±0.4; about ±0.3; about ±0.2; about ±0.1; about ±0.05; or less.
In yet another example, the crystalline form of an L-malic acid salt of Compound I exhibits an XRPD comprising peaks shown in the table below:

**Table 10. XRPD Table of an L-Malic Acid Salt of Compound I**

| **2Theta** | **Flex Width** | **d-value** | **Intensity** | **I/Io** |
|---|---|---|---|---|
| 6.580 | 0.188 | 13.4219 | 1468 | 78 |
| 6.780 | 0.212 | 13.0264 | 1476 | 79 |
| 8.800 | 0.259 | 10.0403 | 959 | 51 |
| 11.800 | 0.259 | 7.4935 | 965 | 52 |
| 13.040 | 0.259 | 6.7836 | 565 | 31 |
| 14.200 | ***** | 6.2320 | 426 | 23 |
| 15.080 | 0.329 | 5.8702 | 561 | 30 |
| 15.940 | 0.353 | 5.5554 | 766 | 41 |
| 16.800 | 0.376 | 5.2729 | 617 | 33 |
| 17.240 | 0.259 | 5.1393 | 675 | 36 |
| 17.660 | 0.165 | 5.0180 | 768 | 41 |
| 18.080 | 0.282 | 4.9024 | 812 | 44 |
| 18.600 | 0.259 | 4.7665 | 963 | 52 |
| 19.560 | 0.329 | 4.5347 | 1207 | 65 |
| 20.160 | 0.165 | 4.4010 | 725 | 39 |
| 20.580 | 0.188 | 4.3121 | 687 | 37 |
| 21.360 | 0.212 | 4.1564 | 539 | 29 |
| 22.020 | 0.212 | 4.0333 | 500 | 27 |
| 22.860 | 0.424 | 3.8870 | 728 | 39 |
| 23.660 | 0.329 | 3.7573 | 1135 | 61 |
| 24.460 | 0.424 | 3.6362 | 1059 | 57 |
| 25.080 | 0.212 | 3.5477 | 1018 | 55 |
| 25.560 | 0.353 | 3.4822 | 1882 | 100 |
| 26.060 | ***** | 3.4165 | 1148 | 61 |
| 26.960 | 0.612 | 3.3044 | 1167 | 63 |
| 27.660 | 0.188 | 3.2224 | 734 | 40 |
| 28.080 | 0.329 | 3.1751 | 785 | 42 |
| 28.740 | 0.353 | 3.1037 | 636 | 34 |
| 29.840 | 0.353 | 2.9917 | 591 | 32 |
| 30.780 | ***** | 2.9025 | 508 | 27 |
| 31.660 | 0.212 | 2.8238 | 553 | 30 |
| 31.900 | 0.188 | 2.8031 | 534 | 29 |

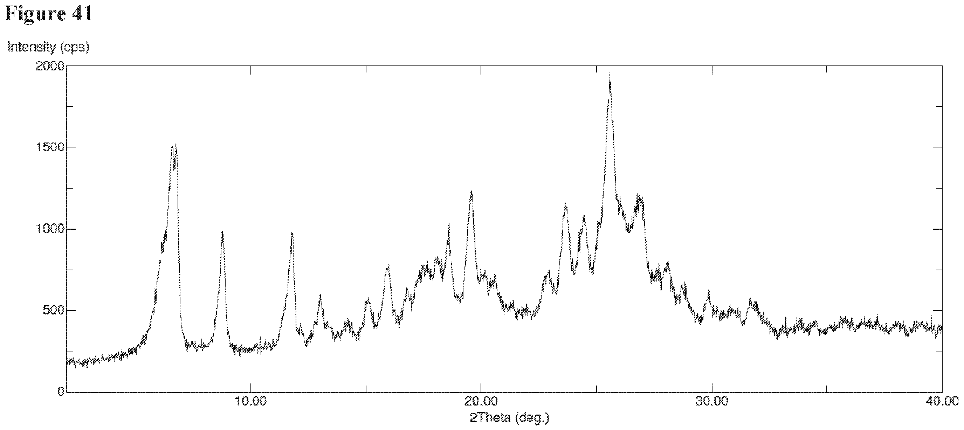

In one specific example, the crystalline form of an L-malic acid salt of Compound I exhibits an XRPD that is substantially similar to Figure 41.

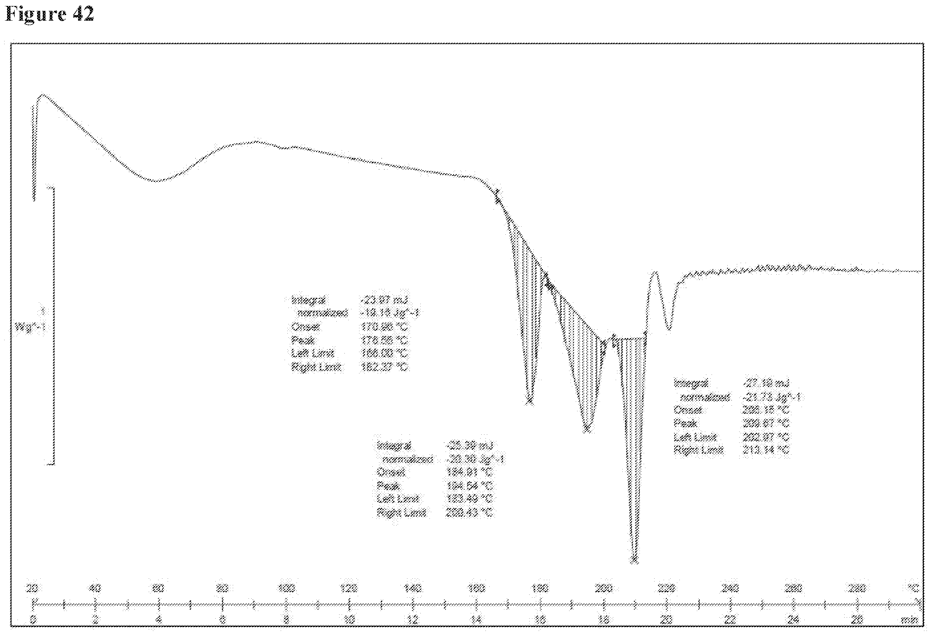

In one example, the crystalline form of an L-malic acid salt of Compound I exhibits a DSC thermogram comprising a peak characteristic value at about 209.67 °C with the error of margin of about ±2.5; about ±2.0; about ±1.5; about ±1.0; about ±0.5; or less. In one specific example, the crystalline form of an L-malic acid salt of Compound I exhibits a DSC thermogram that is substantially similar to Figure 42.

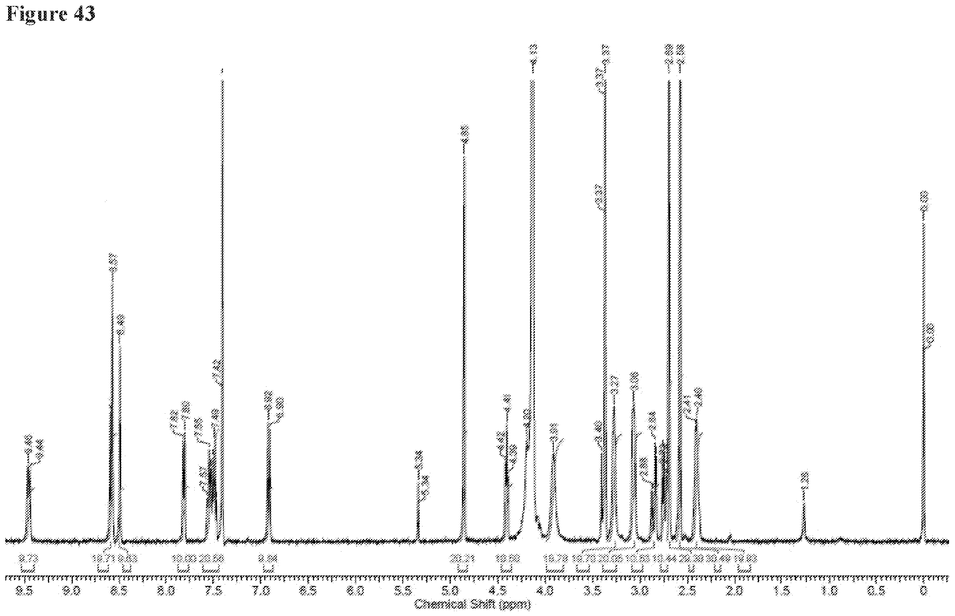

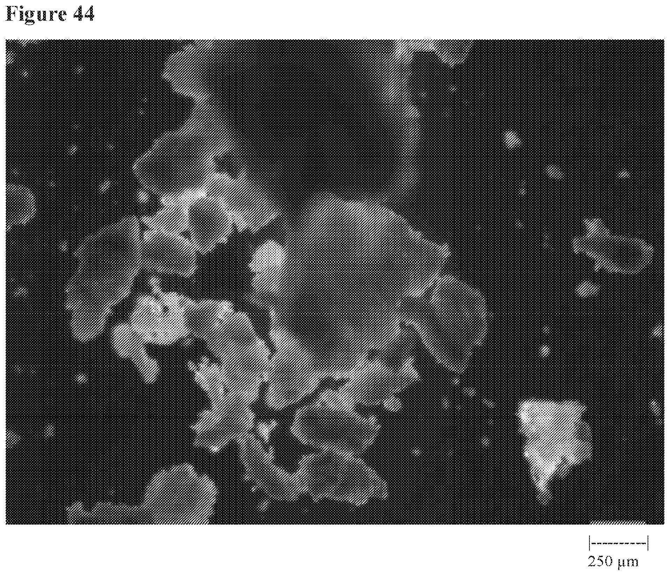

In one example, the crystalline form of an L-malic acid salt of Compound I exhibits a ¹H NMR spectrum that is substantially similar to Figure 43. In another example, the crystalline form of an L-malic acid salt of Compound I may consist of small needles that form agglomerates on a macroscopic scale, which may be substantially similar to Figures 44. In one example, the ratio of L-malic acid and Compound I in the L-malic acid salt of Compound I is about 1: 1.

### Pharmaceutical Formulations

In another example, the present invention provides a pharmaceutical composition comprising a therapeutically effective amount of a crystalline form of Compound I, or a pharmaceutically acceptable salt, ester, and/or solvate thereof, as disclosed herein, as the active ingredient, combined with a pharmaceutically acceptable excipient or carrier. In an embodiment, the present invention provides a pharmaceutical composition comprising a crystalline form of the hydrochloric acid salt of Compound I and at least one pharmaceutically acceptable carrier. The excipients are added to the formulation for a variety of purposes.

Diluents may be added to the formulations of the present invention. Diluents increase the bulk of a solid pharmaceutical composition, and may make a pharmaceutical dosage form containing the composition easier for the patient and care giver to handle. Diluents for solid compositions include, for example, microcrystalline cellulose (e.g., AVICEL), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., EUDRAGIT(r)), potassium chloride, powdered cellulose, sodium chloride, sorbitol, and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g., carbopol), carboxymethylcellulose sodium, dextrin, ethyl cellulose, gelatin, guar gum, gum tragacanth, hydrogenated vegetable oil, hydroxyethyl cellulose, hydroxypropyl cellulose (e.g., KLUCEL), hydroxypropyl methyl cellulose (e.g., METHOCEL), liquid glucose, magnesium aluminum silicate, maltodextrin, methylcellulose, polymethacrylates, povidone (e.g., KOLLIDON, PLASDONE), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g., AC-DI-SOL and PRIMELLOSE), colloidal silicon dioxide, croscarmellose sodium, crospovidone (e.g., KOLLIDON and POLYPLASDONE), guar gum, magnesium aluminum silicate, methyl cellulose, microcrystalline cellulose, polacrilin potassium, powdered cellulose, pregelatinized starch, sodium alginate, sodium starch glycolate (e.g., EXPLOTAB), potato starch, and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance and/or facilitate patient identification of the product and unit dosage level.

In liquid pharmaceutical compositions may be prepared using the crystalline forms of the present invention and any other solid excipients where the components are dissolved or suspended in a liquid carrier such as water, vegetable oil, alcohol, polyethylene glycol, propylene glycol, or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, and xanthan gum.

Sweetening agents such as aspartame, lactose, sorbitol, saccharin, sodium saccharin, sucrose, aspartame, fructose, mannitol, and invert sugar may be added to improve the taste.

Preservatives and chelating agents such as alcohol, sodium benzoate, butylated hydroxyl toluene, butylated hydroxyanisole, and ethylenediamine tetraacetic acid may be added at levels safe for ingestion to improve storage stability.

A liquid composition may also contain a buffer such as guconic acid, lactic acid, citric acid or acetic acid, sodium guconate, sodium lactate, sodium citrate, or sodium acetate. Selection of excipients and the amounts used may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and lozenges, as well as liquid syrups, suspensions, aerosols and elixirs.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

A composition for tableting or capsule filling may be prepared by wet granulation. In wet granulation, some or all of the active ingredients and excipients in powder form are blended and then further mixed in the presence of a liquid, typically water that causes the powders to clump into granules. The granulate is screened and/or milled, dried and then screened and/or milled to the desired particle size. The granulate may be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting; however, they are not subjected to a final tableting step.

The active ingredient and excipients may be formulated into compositions and dosage forms according to methods known in the art.

In one embodiment, a dosage form may be provided as a kit comprising crystalline form of Compound I and pharmaceutically acceptable excipients and carriers as separate components. In some embodiments, the dosage form kit allow physicians and patients to formulate an oral solution or injection solution prior to use by dissolving, suspending, or mixing the crystalline form of Compound I with pharmaceutically acceptable excipients and carriers. In one embodiment, a dosage form kit which provides crystalline form of Compound I has improved stability of Compound I compared to pre-formulated liquid formulations of Compound I.

It is not necessary that the formulations of the present disclsoure contain only one crystalline form of Compound I. The crystalline forms of the present disclsoure may be used in pharmaceutical formulations or compositions as single components or mixtures together with other crystalline forms of Compound I. In one example, pharmaceutical formulations or compositions of the present disclosure contain 25-100% or 50-100% by weight, of at least one of crystalline form of Compound I as described herein, in the formulation or composition.

### Therapeutic Use

The references to the methods of treatment set out below are to be interpreted as references to compounds and/or pharmaceutical compositions of the present invention or disclosure for use in those methods.

The present disclosure also provides treatment of disorders related to proliferation of cells. In one example, there is provided a method for selectively activating p53 protein comprising contacting a cell afflicted by disorder related to cell proliferation with the present compound. In one example, the method comprises contacting cancer and/or tumor cells with the crystalline form of Compound I, or a pharmaceutically acceptable salt, ester, and/or solvate thereof, as disclosed herein. In another example, the method of contacting cancer and/or tumor cells with the crystalline form of Compound I, or a pharmaceutically acceptable salt, ester, and/or solvate thereof, as disclosed herein, may induce cell apoptosis or alleviate or prevent the progression of the disorder.

Additionally, disclosed are methods for treating cancers, cancer cells, tumors, or tumor cells. Non limiting examples of cancer that may be treated by the methods of this disclosure include cancer or cancer cells of: colorectum, breast, lung, liver, pancreas, lymph node, colon, prostate, brain, head and neck, skin, ovary, cervical, thyroid, bladder, kidney, and blood and heart (e.g., leukemia, lymphoma, and carcinoma). Non limiting examples of tumors that may be treated by the methods of this disclosure include tumors and tumor cells of: colorectum, breast, lung, liver, pancreas, lymph node, colon, prostate, brain, head and neck, skin, kidney, and blood and heart (e.g., leukemia, lymphoma, and carcinoma).

The present disclosure also provides methods of treating, preventing, ameliorating and/or alleviating the progression of disorders or conditions characterized by cell proliferation in a subject. More particularly, the methods of the present diclosure involve administration of an effective amount of the crystalline form of the quinolone compounds described herein, in a subject to treat a disorder or a condition characterized by cell proliferation. The crystalline form can be administered in an amount effective selectively activate p53 proteins in cancer and/or tumor cells, which may lead to cell death or apoptosis. The terms "subject" and "patient" are used interchangeably throughout the present application.

As used herein, administering can be effected or performed using any of the various methods known to those skilled in the art. The crystalline form can be administered, for example, subcutaneously, intravenously, parenterally, intraperitoneally, intradermally, intramuscularly, topically, enteral (e.g., orally), rectally, nasally, buccally, sublingually, vaginally, by inhalation spray, by drug pump or via an implanted reservoir in dosage formulations containing conventional non-toxic, physiologically acceptable carriers or vehicles.

Further, the presently disclosed crystalline forms can be administered to a localized area in need of treatment. This can be achieved by, for example, and not by way of limitation, local infusion during surgery, topical application, transdermal patches, by injection, by catheter, by suppository, or by implant (the implant can optionally be of a porous, non-porous, or gelatinous material), including membranes, such as sialastic membranes or fibers.

The form in which the crystalline form is administered (e.g., syrup, elixir, capsule, tablet, foams, emulsion, gel, etc.) will depend in part on the route by which it is administered. For example, for mucosal (e.g., oral mucosa, rectal, intestinal mucosa, bronchial mucosa) administration, nose drops, aerosols, inhalants, nebulizers, eye drops or suppositories can be used. The crystalline form can also be used to coat bioimplantable materials to enhance neurite outgrowth, neural survival, or cellular interaction with the implant surface. The crystalline forms disclosed herein can be administered together with other biologically active agents, such as anticancer agents, analgesics, anti-inflammatory agents, anesthetics and other agents which can control one or more symptoms or causes of a disorder or a condition characterized by cell proliferation.

In one example, the crystalline form of Compound I, or the crystalline form of pharmaceutically acceptable salt, ester, and/or solvate of Compound I, as disclosed herein, can be administered in combination with one or more therapeutically active agent. In one example, the one or more therapeutically active agent is an anticancer agent. In some examples, the one or more therapeutically active anticancer agents include, but are not limited to, paclitaxel, vinblastine, vincristine, etoposide, doxorubicin, herceptin, lapatinib, gefitinib, erlotinib, tamoxifen, fulvestrant, anastrazole, lectrozole, exemestane, fadrozole, cyclophosphamide, taxotere, melphalan, chlorambucil, mechlorethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), doxorubicin (adriamycin), daunorubicin, idarubicin, mitoxantrone, plicamycin, mitomycin, C Bleomycin, combinations thereof, and the like. In another example, the one or more therapeutically active anticancer agents include, but are not limited to, PARP (poly (DP-ribose)polymerase) inhibitors. Suitable PARP inhibitors include, but are not limited to, 4-(3-(1-(cyclopropanecarbonyl)piperazine-4-carbonyl)-4-fluorobenzyl)phthalazin-1(2H)-one (olaparib, AZD2281, Ku-0059436), 2-[(2R)-2-methylpyrrolidin-2-yl]-1H-benzimidazole-4-carboxamide (Veliparib, ABT-888), (8S,9R)-5-fluoro-8-(4-fluorophenyl)-9-(1-methyl-1H-1,2,4-triazol-5-yl)-8,9-dihydro-2H-pyrido[4,3,2-de]phthalazin-3(7H)-one (talazoparib, BMN 673), 4-iodo-3-nitrobenzamide (iniparib, BSI-201), 8-fluoro-5-(4-((methylamino)methyl)phenyl)-3,4-dihydro-2H-azepino[5,4,3-cd]indol-1(6H)-one phosphoric acid (Rucaparib, AG-014699, PF-01367338), 2-[4-[(dimethylamino)methyl]phenyl]-5,6-dihydroimidazo[4,5,1-*jk*][1,4]benzodiazepin-7(4H)-one (AG14361), 3-aminobenzamide (INO-1001), 2-(2-fluoro-4-((S)-pyrrolidin-2-yl)phenyl)-3H-benzo[d]imidazole-4-carboxamide (A-966492), N-(5,6-dihydro-6-oxo-2-phenanthridinyl)-2-acetamide hydrochloride (PJ34, PJ34 HCl), MK-4827, 3,4-dihydro-4-oxo-3,4-dihydro-4-oxo-N-[(1S)-1-phenylethyl]-2-quinazolinepropanamide (ME0328), 5-(2-oxo-2-phenylethoxy)-1(2H)-isoquinolinone (UPF-1069), 4-[[4-fluoro-3-[(4-methoxy-1-piperidinyl)carbonyl]phenyl]methyl]-1(2H)-phthalazinone (AZD 2461), and the like. In another example, the one or more therapeutically active agent is an immunotherapeutic agent. In some examples, the one or more immunotherapeutic agents includes, but are not limited to, a monoclonal antibody, an immune effector cell, adoptive cell transfer, an immunotoxin, a vaccine, a cytokine, and the like.

In another example, the crystalline form of Compound I, or the crystalline form of pharmaceutically acceptable salt, ester, and/or solvate of Compound I, as disclosed herein, can be administered in combination with radiotherapy.

Additionally, administration can comprise administering to the subject a plurality of dosages over a suitable period of time. Such administration regimens can be determined according to routine methods, upon a review of the instant disclosure.

Crystalline forms of the invention are generally administered in a dose of about 0.01 mg/kg/dose to about 100 mg/kg/dose. Alternately the dose can be from about 0.1 mg/kg/dose to about 10 mg/kg/dose; or about 1 mg/kg/dose to 10 mg/kg/dose. Time release preparations may be employed or the dose may be administered in as many divided doses as is convenient. When other methods are used (e.g. intravenous administration), crystalline forms are administered to the affected tissue at a rate from about 0.05 to about 10 mg/kg/hour, alternately from about 0.1 to about 1 mg/kg/hour. Such rates are easily maintained when these crystalline forms are intravenously administered as discussed herein. Generally, topically administered formulations are administered in a dose of about 0.5 mg/kg/dose to about 10 mg/kg/dose range. Alternately, topical formulations are administered at a dose of about 1 mg/kg/dose to about 7.5 mg/kg/dose or even about 1 mg/kg/dose to about 5 mg/kg/dose.

A range of from about 0.1 to about 100 mg/kg is appropriate for a single dose. Continuous administration is appropriate in the range of about 0.05 to about 10 mg/kg.

Drug doses can also be given in milligrams per square meter of body surface area rather than body weight, as this method achieves a good correlation to certain metabolic and excretionary functions. Moreover, body surface area can be used as a common denominator for drug dosage in adults and children as well as in different animal species (Freireich et al., (1966) Cancer Chemother Rep. 50, 219-244). Briefly, to express a mg/kg dose in any given species as the equivalent mg/sq m dose, the dosage is multiplied by the appropriate km factor. In an adult human, 100 mg/kg is equivalent to 100 mg/kgx37 kg/sq m=3700 mg/m².

A dosage form of the present disclosure may contain Compound I, or a pharmaceutically acceptable salt, ester, and/or solvate thereof, as disclosed herein, in an amount of about 5 mg to about 500 mg. That is, a dosage form of the present invention may contain Compound I in an amount of about 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 175 mg, 180 mg, 190 mg, 200 mg, 210 mg, 220 mg, 225 mg, 230 mg, 240 mg, 250 mg, 260 mg, 270 mg, 275 mg, 280 mg, 290 mg, 300 mg, 310 mg, 320 mg, 325 mg, 330 mg, 340 mg, 350 mg, 360 mg, 370 mg, 375 mg, 380 mg, 390 mg, 400 mg, 410 mg, 420 mg, 425 mg, 430 mg, 440 mg, 450 mg, 460 mg, 470 mg, 475 mg, 480 mg, 490 mg, or 500 mg. In one embodiment, such dosage amount is administered to a patient as a daily dose either in a single dose or in divided portions served multiple times a day, such as twice, three times, or four times a day.

A dosage form of the present invention may be administered, hourly, daily, weekly, or monthly. The dosage form of the present invention may be administered twice a day or once a day. The dosage form of the present invention may be administered with food or without food.

Insofar as the crystalline forms disclosed herein can take the form of a mimetic or fragment thereof, it is to be appreciated that the potency, and therefore dosage of an effective amount can vary. However, one skilled in the art can readily assess the potency of a crystalline form of the type presently envisioned by the present application.

In settings of a gradually progressive disorder or condition characterized by cell proliferation, crystalline forms of the present application are generally administered on an ongoing basis. In certain settings administration of a crystalline form disclosed herein can commence prior to the development of disease symptoms as part of a strategy to delay or prevent the disease. In other settings a crystalline form disclosed herein is administered after the onset of disease symptoms as part of a strategy to slow or reverse the disease process and/or part of a strategy to improve cellular function and reduce symptoms.

It will be appreciated by one of skill in the art that dosage range will depend on the particular crystalline form, and its potency. The dosage range is understood to be large enough to produce the desired effect in which the neurodegenerative or other disorder and the symptoms associated therewith are ameliorated and/or survival of the cells is achieved, but not be so large as to cause unmanageable adverse side effects. It will be understood, however, that the specific dose level for any particular patient will depend on a variety of factors including the activity of the specific crystalline form employed; the age, body weight, general health, sex and diet of the individual being treated; the time and route of administration; the rate of excretion; other drugs which have previously been administered; and the severity of the particular disease undergoing therapy, as is well understood by those skilled in the art. The dosage can also be adjusted by the individual physician in the event of any complication. No unacceptable toxicological effects are expected when crystalline forms disclosed herein are used in accordance with the present application.

An effective amount of the crystalline forms disclosed herein comprise amounts sufficient to produce a measurable biological response. Actual dosage levels of active ingredients in a therapeutic crystalline form of the present application can be varied so as to administer an amount of the active crystalline form that is effective to achieve the desired therapeutic response for a particular subject and/or application. Preferably, a minimal dose is administered, and the dose is escalated in the absence of dose-limiting toxicity to a minimally effective amount. Determination and adjustment of a therapeutically effective dose, as well as evaluation of when and how to make such adjustments, are known to those of ordinary skill in the art.

Further with respect to the methods of the present application, a preferred subject is a vertebrate subject. A preferred vertebrate is warm-blooded; a preferred warm-blooded vertebrate is a mammal. The subject treated by the presently disclosed methods is desirably a human, although it is to be understood that the principles of the present application indicate effectiveness with respect to all vertebrate species which are included in the term "subject." In this context, a vertebrate is understood to be any vertebrate species in which treatment of a neurodegenerative disorder is desirable. As used herein, the term "subject" includes both human and animal subjects. Thus, veterinary therapeutic uses are provided in accordance with the present application.

As such, the present application provides for the treatment of mammals such as humans, as well as those mammals of importance due to being endangered, such as Siberian tigers; of economic importance, such as animals raised on farms for consumption by humans; and/or animals of social importance to humans, such as animals kept as pets or in zoos or farms. Examples of such animals include but are not limited to: carnivores such as cats and dogs; swine, including pigs, hogs, and wild boars; ruminants and/or ungulates such as cattle, oxen, sheep, giraffes, deer, goats, bison, and camels; and horses. Also provided is the treatment of birds, including the treatment of those kinds of birds that are endangered and/or kept in zoos, as well as fowl, and more particularly domesticated fowl, i.e., poultry, such as turkeys, chickens, ducks, geese, guinea fowl, and the like, as they are also of economical importance to humans. Thus, also provided are the treatment of livestock, including, but not limited to, domesticated swine, ruminants, ungulates, horses (including race horses), poultry, and the like.

The following examples further illustrate the present disclosure but should not be construed as in any way limiting its scope.

### EXAMPLES

**Analytical Methods** - various analytical methods, as described below, were applied to the present crystalline forms and their precursors to characterize their physiochemical properties.

### DIFFERENTIAL SCANNING CALORIMETRY (DSC):

DSC data were collected on DSC-systems (DSC 822e - Mettler Toledo) or on TA Instruments Q2000. In general, samples in the mass range of 1 to 5 mg were placed in a dry aluminum crucible and closed with an aluminum cap with a hole. Generally, the starting temperature was 20 °C, the heating rate was 10 °C/min, and final temperature was 300 °C using a nitrogen purge flow of 20 mL/min.

### THERMOGRAVIMETRIC ANALYSIS (TGA):

TGA data were collected on a TGA 851e apparatus. In general, samples in the mass range of approximately10 mg were placed in a lean and dry aluminum oxide pan or an aluminum pan. Generally, the sample pan and scanned between -30 °C to about 350 °C at 5 °C/minute or at 10 °C/minute using a nitrogen purge flow rate at 50 mL/min.

TGA analysis was also performed using a TA Instruments Discovery IR thermogravimetric analyzer. Temperature calibration was performed using nickel and Alumel^{™}. The sample was placed in an aluminum pan. The sample was hermetically sealed, the lid pierced, then inserted into the thermogravimetric furnace. The furnace was heated under nitrogen. The sample was analyzed from ambient to 350 °C with a heating rate of 10 °C/min. Trios software v3.1.2.3591 was used to generate thermograms.

### ¹H NUCLEAR MAGNETIC RESONANCE SPECTROSCOPY (¹H NMR):

1H NMR spectra was obtained using a Bruker AVANCE 400 MHz instrument in DMSO-d₆ or CDCl₃ with tetramethylsilane as the internal standard.

### LIGHT MICROSCOPY WITH HOT STAGE (HSM):

An Olympus BX41 with Di-Li 5MP camera and grab&measure software was used with Hostage Mettler Toledo FP90 with FP 82 heating table. Samples were prepared with brushes onto object holders. Observation was made using unpolarized light or polarized light using two polarization filters at 40, 100, 200 or 400 x magnifications. Pictures were taken by software and exported as JPEG, scale is only approximate and not validated.

### X-RAY POWDER DIFFRACTION (XRPD):

XRPD patterns were obtained using a MiniFlex (Rigaku Corporation) using silicon low background sample holders (diameter 24 mm, pit 0.2 mm). Collection times were nominally 75 minutes. A Cu Kα radiation 1.5406 angstrom source operating at 15 kV was used to irradiate samples. The effective 2θ range was about 2-40 °C and the sampling width was 0.02 °C. Samples were ground with mortar and pestle. Solids were positioned on sample holders prepared with grease and flattened with a disc of glass.

### HPLC ANALYSIS:

Crystalline forms (i.e., salts and free base) of the present invention were analyzed by total area normalization (TAN).

### HPLC Conditions:

| | |
|---|---|
| HPLC Column: | Phenomenex Luna8, 3 µm C18, 4.6 × 50 mm |
| Flow Rate: | 1.0 mL/min |
| Injection Volume: | 5 µL |
| Detection: | DAD detector, recording at 240 nm |
| Mobile Phase: | A - H₂O + 0.05% CF₃COOH |
| | B - CAN + 0.05% CF₃COOH |

### Gradient Pump Program:

| Step Time (minutes) | % A | % B |
|---|---|---|
| 0.00 | 90.0 | 10.0 |
| 0.10 | 90.0 | 10.0 |
| 10.1 | 10.0 | 90.0 |
| 12.1 | 10.0 | 90.0 |
| 15.0 | 90.0 | 10.0 |

### Example 1. Solubility of 2-(4-Methyl-[1,4]diazepan-1-yl)-5-oxo-5H-7-thia-1,11b-diaza-benzo[c]fluorene-6-carboxylic acid (5-methyl-pyrazin-2-ylmethyl)-amide (Compound I, free base):

Solubility of Compound I was determined by suspending Compound I in various solvents then heated at an elevated temperature then cooled to room temperature. The mother liquor was sampled and the concentration of Compound I was determined by HPLC.

### Example 2. Preparation of polymorph E of Compound I (free base):

Compound I (free base) was dissolved in a solvent mixture to form a solution, which was then concentrated into a suspension. The suspension was then concentrated to dryness. An anti-solvent was added to the suspension, which was concentrated to dryness again. XRPD analysis confirmed formation of polymorph E and revealed polymorph E as crystalline (Fig. 11). The dry content was measured to be 99.97% w/w. ¹H NMR (Fig. 13) confirmed the structure of this material as the free base of Compound.

### Example 3. Preparation of polymorph A of Compound I (free base):

Polymorph E of Compound I (free base) as prepared by Example 2 was suspended in a solvent mixture. The suspension was heated and then cooled down to room temperature. The suspension was filtered, and the resulting filter cake was dried to provide polymorph A. XRPD analysis confirmed formation of polymorph A and revealed polymorph A as crystalline (Fig. 1). The dry content was measured to be 99.86% w/w. ¹H NMR (Fig. 5) confirmed the structure of this material as the free base of Compound I.

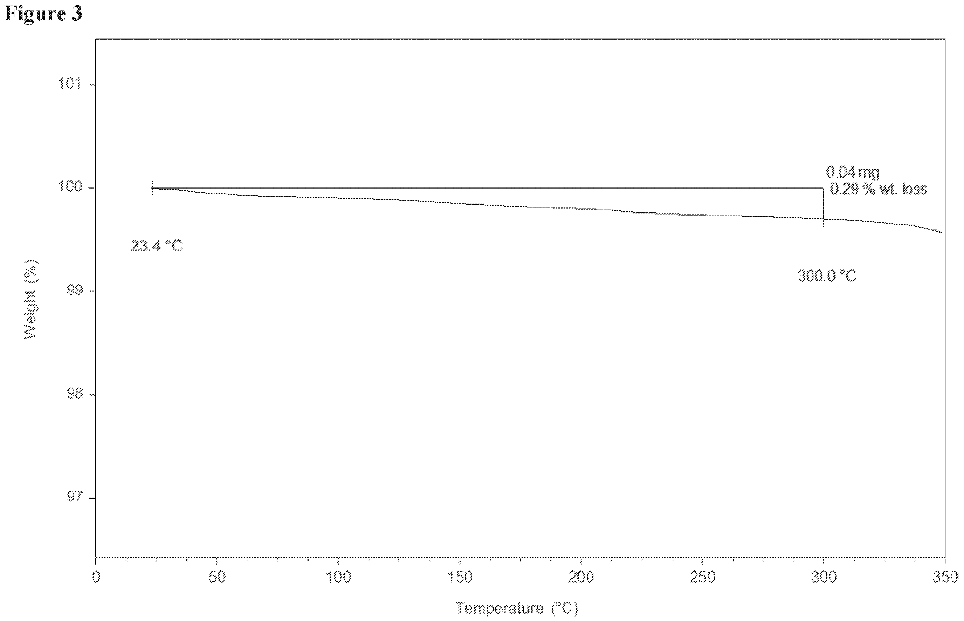

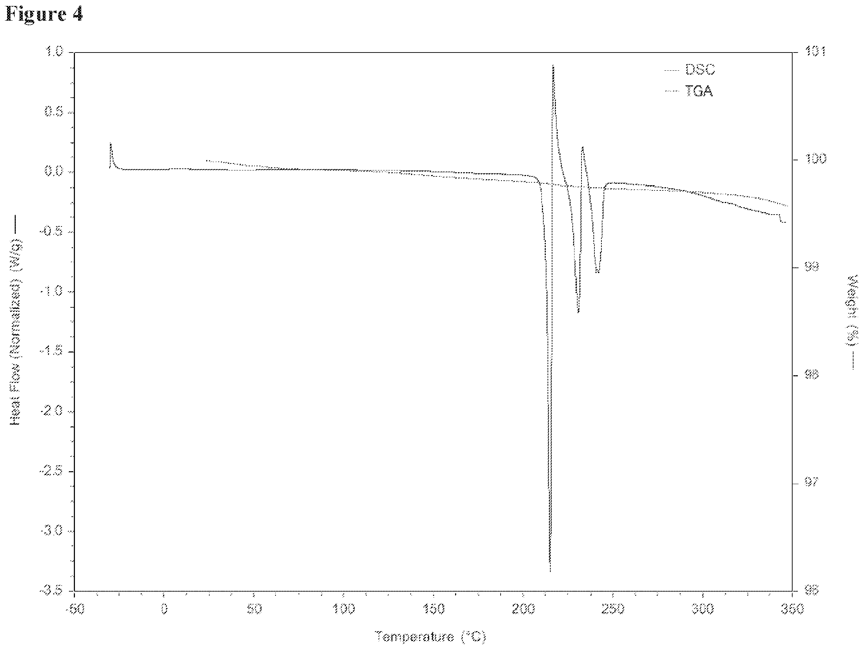

The DSC thermogram (Fig. 2) exhibits a sharp endotherm at approximately 215 °C, followed by an exotherm at approximately 217 °C, an endotherm at approximately 231 °C, an exotherm at approximately 233 °C, and an endotherm at approximately 242 °C. Without bound to any theory, these events are consistent with a melt, followed by a recrystallization, another melt and recrystallization, and a final melt event. TGA thermogram (Fig. 3) displays an approximate 0.3% weight loss from ambient to 300 °C. Figure 4 shows an overlay of the DSC and TGA thermograms.

### Example 4. Preparation of polymorph C of Compound I (free base):

Polymorph A of Compound I was suspended in an organic solvent at an elevated temperature, filtered and the mother liquor was cooled to form a suspension, which was filtered to obtain white needle like solids. ¹H NMR (Fig. 9) confirmed the structure of this material as the free base of Compound I.

XRPD analysis revealed polymorph C as crystalline, as shown in Figure 7.

### Example 5. Preparation of polymorph G of Compound I (free base):

Polymorph A of Compound I was suspended in another organic solvent at an elevated temperature. The suspension was filtered and the mother liquor was evaporated to form a suspension, which was filtered to obtain slightly yellow solid. ¹H NMR (Fig. 18) confirmed the structure of this material as the free base of Compound I.

XRPD analysis revealed polymorph G as crystalline, as shown in Figure 16.

### Example 6. Solubility and Stability test of polymorph forms

Solubility of polymorphs A, C, E, and G of Compound I (free base) were determined by suspending approximately 25 mg of each polymorph in organic solvents and the resulting mixtures were stirred at room temperature or elevated temperatures for 1 h. A sample was filtered and the concentration was determined by HPLC. The remaining suspension was evaporated and the solid was analyzed by XRPD to confirm that the polymorph form in the suspension was still the same polymorph form used for the experiment.

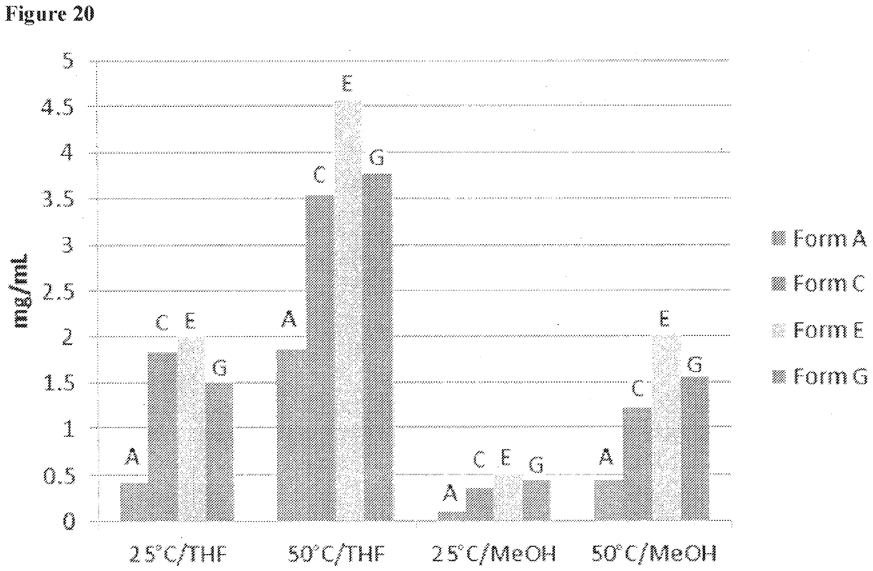

The solubility of each polymorph is shown in Figure 20. Polymorph A exhibited the lowest solubility, identifying it as the most stable.

### Example 7. Preparation of a HCl Salt of Compound I:

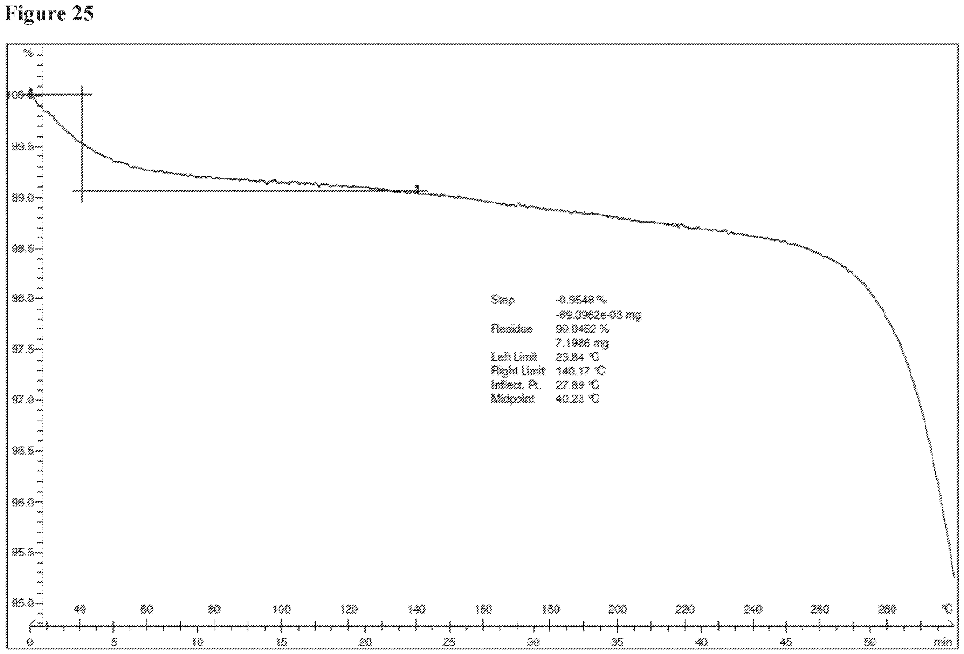

Compound I was dissolved in a solvent mixture at an elevated temperature, then HCl was added. The suspension was stirred overnight then filtered and washed to obtain an HCl salt of Compound I as a white powder (62% yield). The resulting salt was characterized by ¹H NMR (Fig. 23), XRPD (Fig. 21), and TGA (Fig. 25). The TGA showed HCl salt of Compound I demonstrated up to 0.9 wt% loss at 140 °C.

### Example 8. Preparation of a Maleic Acid Salt of Compound I:

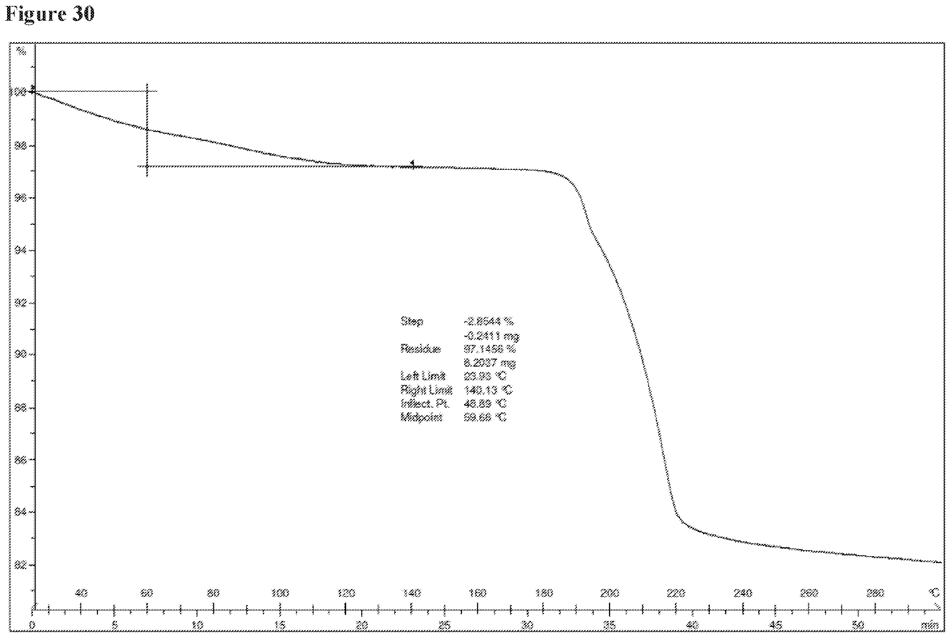

Compound I was dissolved in a solvent mixture at an elevated temperature, then Maleic acid was added to the mixture. The resulting suspension was cooled and stirred, filtered, and washed to obtain a maleic acid salt of Compound I as a white powder (86% yield). The resulting salt was characterized by ¹H NMR (Fig. 28), XRPD (Fig. 26), and TGA (Fig. 30). The TGA showed maleic acid salt of Compound I demonstrated up to 2.9 wt% loss at 140 °C. Without being bound by any theory, this weight loss may be attributed to solvent loss.

### Example 9. Preparation of a Fumaric Acid Salt of Compound I:

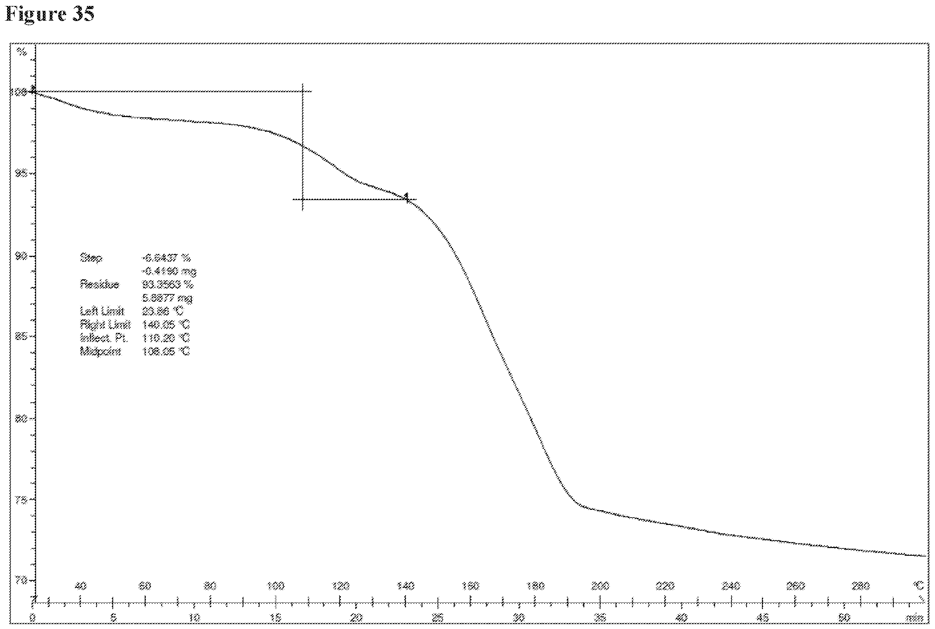

Compound I was dissolved in a solvent mixture at an elevated temperature, then Fumaric acid was added to the mixture. The resulting suspension was cooled, stirred, filtered, and washed to obtain a fumaric acid salt of Compound I as a white powder (104% yield). The resulting salt was characterized by ¹H NMR (Fig. 33), XRPD (Fig. 31), and TGA (Fig. 35). The TGA showed fumaric acid salt of Compound I demonstrated up to 6.6% weight loss at 140 °C.

### Example 10. Preparation of a Citric Acid Salt of Compound I:

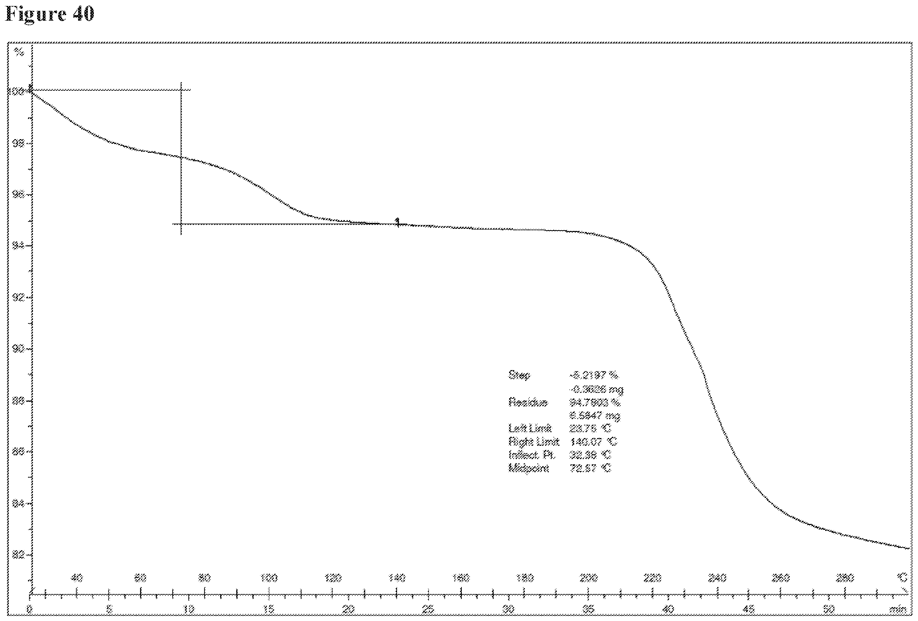

Compound I was dissolved in a solvent mixture at an elevated temperature, then Citric acid was added to the mixture. The resulting suspension was cooled, stirred, filtered, and washed to obtain a citric acid salt of Compound I as a white powder (93% yield). The resulting salt was characterized by ¹H NMR (Fig. 38), XRPD (Fig. 36), and TGA (Fig. 40). The TGA showed citric acid salt of Compound I demonstrated up to 5.2% weight loss at 140 °C.

### Example 11. Preparation of an L-Malic Acid Salt of Compound I:

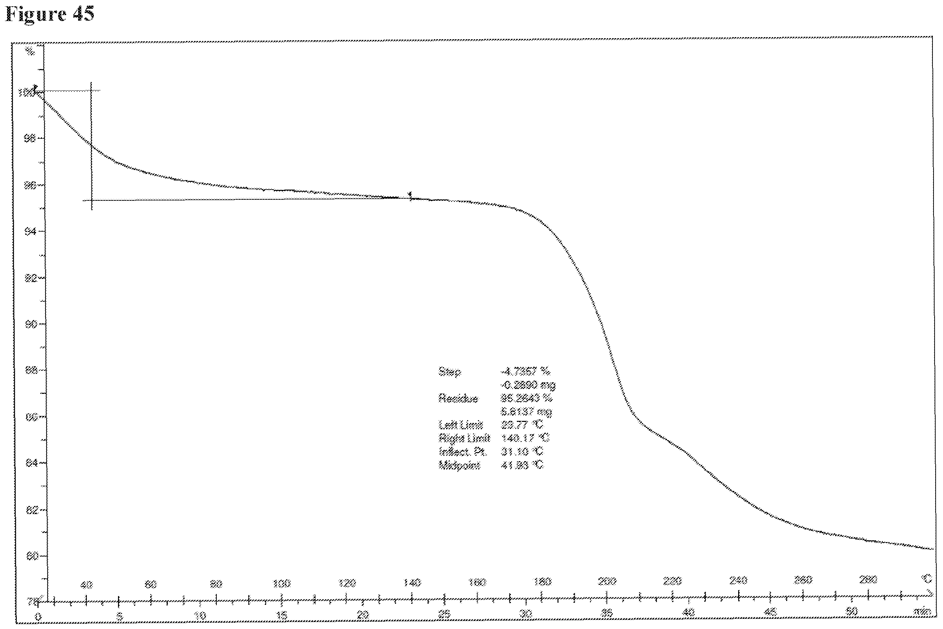

Compound I was dissolved in a solvent mixture at an elevated temperature, then L-Malic acid was added to the mixture. The resulting suspension was cooled, stirred, filtered, and washed to obtain an L-malic acid salt of Compound I as a white powder (59% yield). The resulting salt was characterized by ¹H NMR (Fig. 43), XRPD (Fig. 41), and TGA (Fig. 45). The TGA showed citric acid salt of Compound I demonstrated up to 4.7% weight loss at 140 °C.

### Example 12. Solubility and Stability Test of Acid Salts of Compound I

Solubility of the HCl salt, maleic acid salt, fumaric acid salt, citric acid salt, and L-malic acid salt of Compound I were determined by suspending approximately 50 mg of each salt in 0.25 mL water (x 2, added portion wise). The mixture was stirred at 42 °C for 3 days and each solution was analyzed by HPLC for concentration and purity. The solubility and purity of each salt after 3 day test are shown in Table 11 below.

**Table 11 Solubility and Purity of Acid Addition Salts**

| **Acid Salt** | **Aqueous Solubility (Calculated)** | **Starting Purity % a/a (HPLC)** | **Purity % a/a after 3 days (HPLC)** |
|---|---|---|---|
| HCl | > 100 mg/mL | 98.78 | 88.62 |
| Maleic Acid | > 200 mg/mL | 99.18 | 83.87 |
| Fumaric Acid | < 100 mg/mL* | 98.46 | 82.38 |
| Citric Acid | > 200 mg/mL | 98.62 | 95.21 |
| L-Malic Acid | < 100 mg/mL | 98.07 | 95.08 |
| Free Base | << 100 mg/mL | NT | 99.58 |

| | | | |
|---|---|---|---|
| ^{∗}Sample dissolved during 3 day stability test; NT = not tested | | | |

Stability of the acid salts were also tested by storing the salts (solid forms) at an elevated temperature of 45 and 80 °C. The results of the stability test are shown in Table 12, below.

**Table 12 Stability of Acid Addition Salts**

| **Acid Salt** | **Purity % a/a after 3 days at 80 °C (HPLC)** | **Purity % a/a after 3 days at 45 °C (HPLC)** |
|---|---|---|
| HCl | 98.71 | 98.48 |
| Maleic Acid | 93.67 | 97.84 |
| Fumaric Acid | 89.36 | NT |
| Citric Acid | 95.17 | NT |
| L-Malic Acid | 95.67 | NT |
| Free Base | 99.67 | NT |

| | | |
|---|---|---|
| NT = not tested | | |

## Claims

1. A crystalline form of a Compound I: 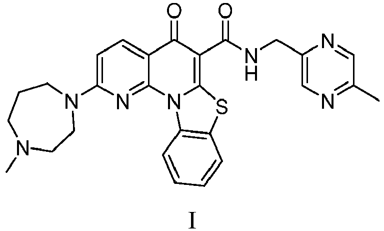 or a pharmaceutically acceptable salt thereof, wherein the crystalline form is hydrochloric acid salt of Compound I.

2. A composition comprising a crystalline form of claim 1 and at least one pharmaceutically acceptable carrier.

3. A crystalline form of claim 1 or 2 for use in treating or ameliorating cell proliferation disorder, wherein the cell proliferation disorder is cancer selected from: heme cancer, colorectum cancer, breast cancer, lung cancer, liver cancer, ovarian cancer, cervical cancer, Ewing's sarcoma, pancreatic cancer, cancer of the lymph nodes, colon cancer, prostate cancer, brain cancer, cancer of the head and neck, skin cancer, kidney cancer, or cancer of the heart.

4. A crystalline form of claim 1 or 2 for use in reducing or inhibiting cell proliferation, wherein the cells are in cancer cells selected from: heme cancer, colorectum cancer, breast cancer, lung cancer, liver cancer, ovarian cancer, cervical cancer, Ewing's sarcoma, pancreatic cancer, cancer of the lymph nodes, colon cancer, prostate cancer, brain cancer, cancer of the head and neck, skin cancer, kidney cancer, or cancer of the heart.

5. The crystalline form for use of claim 3 or 4, wherein the heme cancer is selected from leukemia, lymphoma, myeloma, or multiple myeloma.

6. The crystalline form for use of any one of claims 3-5, wherein
(a) the cancer is homologous recombination (HR) dependent double strand break (DSB) repair deficient cancer or non-homologous end joining (NHEJ) DSB repair deficient cancer; or
(b) the cancer comprises cancer cells harboring defects in breast cancer 1 (BRCA1), breast cancer 2 (BRCA2), and/or other members of the homologous recombination pathway.

7. The crystalline form for use of claim 6, wherein the cancer comprises cancer cells deficient in BRCA1 and/or BRCA2.

8. The crystalline form for use of claim 7, wherein
(a) the cancer cells are homozygous for a mutation in BRCA1 and/or BRCA2; or
(b) the cancer cells are heterozygous for a mutation in BRCA1 and/or BRCA2.

9. The crystalline form for use of any one of claims 3, 4 and 6-8, wherein the cancer is breast cancer.

10. An HCl salt of Compound I

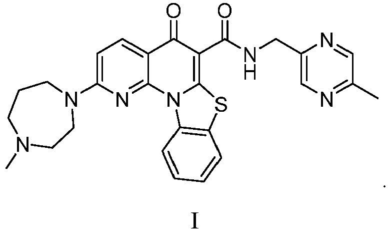

## Patentansprüche

1. Kristalline Form einer Verbindung I: 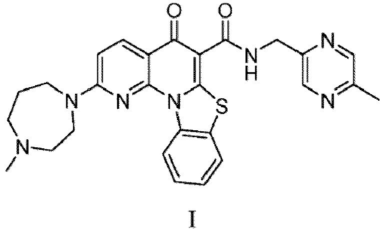 oder pharmazeutisch unbedenkliches Salze einer solchen, wobei es sich bei der kristallinen Form um ein Salzsäuresalz der Verbindung I handelt.

2. Zusammensetzung, die eine kristalline Form nach Anspruch 1 und mindestens einen pharmazeutisch unbedenklichen Trägerstoff umfasst.

3. Kristalline Form nach Anspruch 1 oder 2 zur Verwendung in der Behandlung oder Linderung einer Zellvermehrungsstörung, wobei es sich bei der Zellvermehrungsstörung um eine Krebserkrankung handelt, die aus den folgenden ausgewählt ist: hämbasierten Krebserkrankungen, dem kolorektalen Karzinom, Brustkrebs, Lungenkrebs, Leberkrebs, Eierstockkrebs, Gebärmutterhalskrebs, dem Ewing-Sarkom, Bauchspeicheldrüsenkrebs, Krebserkrankungen der Lymphknoten, Dickdarmkrebs, Prostatakrebs, Hirntumoren, Krebserkrankungen im Kopf- und Halsbereich, Hautkrebs, einem Nierenkarzinom oder Krebserkrankungen des Herzens.

4. Kristalline Form nach Anspruch 1 oder 2 zur Verwendung in der Verringerung oder Hemmung einer Zellvermehrung, wobei es sich bei den Zellen um Krebszellen handelt, die aus Folgendem ausgewählt sind: hämbasierten Krebserkrankungen, dem kolorektalen Karzinom, Brustkrebs, Lungenkrebs, Leberkrebs, Eierstockkrebs, Gebärmutterhalskrebs, dem Ewing-Sarkom, Bauchspeicheldrüsenkrebs, Krebserkrankungen der Lymphknoten, Dickdarmkrebs, Prostatakrebs, Hirntumoren, Krebserkrankungen im Kopf- und Halsbereich, Hautkrebs, einem Nierenkarzinom oder Krebserkrankungen des Herzens.

5. Kristalline Form zur Verwendung nach Anspruch 3 oder 4, wobei die hämbasierte Krebserkrankung aus Leukämien, Lymphomen, Myelomen oder dem multiplen Myelom ausgewählt sind.

6. Kristalline Form zur Verwendung nach einem beliebigen der Ansprüche 3 bis 5, wobei
(a) es sich bei der Krebserkrankung um eine Krebserkrankung mit einer unzureichenden Doppelstrangbruch(DSB)-Reparatur, welche von homologer Rekombination (HR) abhängig ist, und um eine Krebserkrankung mit einer unzureichenden DSB-Reparatur handelt, welche durch das Zusammenfügen nicht-homologer Enden (NHEJ) erfolgt; oder
(b) die Krebserkrankung Krebszellen beherbergt, bei denen das Brustkrebsgen 1 (BRCA1), das Brustkrebsgen 2 (BRCA2) und/oder andere Glieder des Stoffwechselwegs der homologen Rekombination schadhaft sind.

7. Kristalline Form zur Verwendung nach Anspruch 6, wobei die Krebserkrankung Krebszellen umfasst, denen es an BRCA1 und/oder BRCA2 fehlt.

8. Kristalline Form zur Verwendung nach Anspruch 7, wobei
(a) die Krebszellen homozygot sind, was eine Mutation in BRCA1 und/oder BRCA2 betrifft; oder
(b) die Krebszellen heterozygot sind, was eine Mutation in BRCA1 und/oder BRCA2 betrifft.

9. Kristalline Form zur Verwendung nach einem beliebigen der Ansprüche 3, 4 und 6 bis 8, wobei es sich bei der Krebserkrankung um Brustkrebs handelt.

10. HCl-Salz der Verbindung I

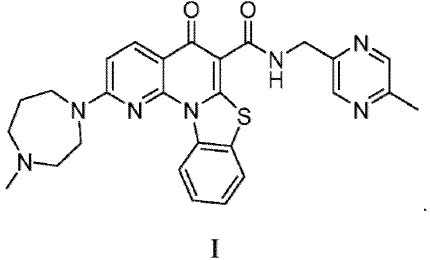

## Revendications

1. Forme cristalline d'un composé I : 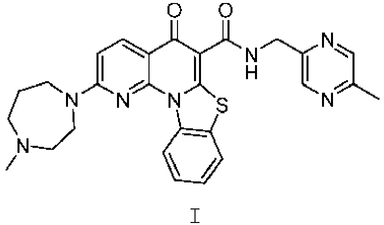 ou un sel pharmaceutiquement acceptable de celui-ci, la forme cristalline étant le sel d'acide chlorhydrique du composé I.

2. Composition comprenant une forme cristalline selon la revendication 1 et au moins un véhicule pharmaceutiquement acceptable.

3. Forme cristalline selon la revendication 1 ou 2 pour utilisation dans le traitement ou l'amélioration d'un trouble de prolifération cellulaire, le trouble de prolifération cellulaire étant un cancer choisi parmi : un cancer hématologique, un cancer colorectal, un cancer du sein, un cancer du poumon, un cancer du foie, un cancer des ovaires, un cancer du col de l'utérus, un sarcome d'Ewing, un cancer du pancréas, un cancer des ganglions lymphatiques, un cancer du côlon, un cancer de la prostate, un cancer du cerveau, un cancer de la tête et du cou, un cancer de la peau, un cancer du rein ou un cancer du coeur.

4. Forme cristalline selon la revendication 1 ou 2 pour utilisation dans la réduction ou l'inhibition de la prolifération cellulaire, les cellules étant des cellules cancéreuses choisies parmi : un cancer hématologique, un cancer colorectal, un cancer du sein, un cancer du poumon, un cancer du foie, un cancer des ovaires, un cancer du col de l'utérus, un sarcome d'Ewing, un cancer du pancréas, un cancer des ganglions lymphatiques, un cancer du côlon, un cancer de la prostate, un cancer du cerveau, un cancer de la tête et du cou, un cancer de la peau, un cancer du rein ou un cancer du coeur.

5. Forme cristalline pour utilisation selon la revendication 3 ou 4, le cancer hématologique étant choisi parmi une leucémie, un lymphome, un myélome ou un myélome multiple.

6. Forme cristalline pour utilisation selon l'une quelconque des revendications 3 à 5, où
(a) le cancer est un cancer déficient en réparation de cassure double brin (DSB) dépendante d'une recombinaison homologue (HR) ou un cancer déficient en réparation de DSB d'assemblage d'extrémités non homologues (NHEJ) ; ou
(b) le cancer comprend des cellules cancéreuses présentant des déficits dans le cancer du sein 1 (BRCA1), le cancer du sein 2 (BRCA2), et/ou d'autres membres de la voie de recombinaison homologue.

7. Forme cristalline pour utilisation selon la revendication 6, le cancer comprenant des cellules cancéreuses déficientes en BRCA1 et/ou BRCA2.

8. Forme cristalline pour utilisation selon la revendication 7, où
(a) les cellules cancéreuses sont homozygotes pour une mutation dans BRCA1 et/ou BRCA2 ; ou
(b) les cellules cancéreuses sont hétérozygotes pour une mutation dans BRCA1 et/ou BRCA2.

9. Forme cristalline pour utilisation selon l'une quelconque des revendications 3, 4 et 6 à 8, le cancer étant un cancer du sein.

10. Sel de HCl du composé I
